# EUROPEAN PATENT SPECIFICATION

(11) **EP 1 551 274 B1**
(45) Date of publication and mention of the grant of the patent: **24.12.2014**
(21) Application number: 03770389.9
(22) Date of filing: 23.09.2003
(51) Int. Cl.: A61F 2/24

(54) **PROSTHETIC MITRAL VALVE**
KÜNSTLICHE MITRALKLAPPE
PROTHESE DE VALVULE MITRALE

(30) Priority: 23.09.2002 US 413266 P
(43) Date of publication of application: 13.07.2005
(73) Proprietor: Medtronic 3F Therapeutics, Inc., Lake Forest CA 92630 (US)
(72) Inventor: CALI, Douglas, - (US); MYERS, Keith, - (US); BIANCUCCI, Brian, - (US); ARTOF, Jason, - (US); NGUYEN, Christine, - (US); QUIJANO, Rodolfo, - (US)
(74) Representative: Zimmermann & Partner
(86) International application number: PCT/US2003/029903
(87) International publication number: WO 2004/026117

(56) References cited:
- US-A- 4 275 469
- US-A- 4 960 424
- US-A- 4 960 424
- US-A- 5 344 442
- US-A- 5 344 442
- US-A1- 2002 052 651
- US-A1- 2002 052 651
- US-B1- 6 358 277

## Description

### Field of the Invention

The present invention relates to an improved prosthetic mitral valve and an apparatus for testing prosthetic mitral valves.

### Backgroung of the Invention

A natural human heart has four valves that serve to direct blood now through the heart. On the right (pulmonary) side of the heart are: (1) the tricuspid valve, which is positioned generally between the right atrium and the right ventricle, and (2) the pulmonary valve, which is positioned generally between the right ventricle and the pulmonary artery. These two valves direct de-oxygenated blood from the body through the right side of the heart and into the pulmonary artery for distribution to the lungs, where the blood is re-oxygenated. On the left (systemic) side of the heart are: (1) the mitral valve, which is positioned generally between the left atrium and the left ventricle, and (2) the aortic valve, which is positioned generally between the left ventricle and the aorta. These two valves direct oxygenated blood from the lungs through the left side of the heart and into the aorta for distribution to the body.

All four of these heart valves are passive structures in that they do not themselves expend any energy and do not perform any active contractile function. They consist of moveable "leaflets" that open and close in response to differential blood pressures on either side of the valve. The mitral and tricuspid valves are referred to as "atrioventricular" valves because they are situated generally between an atrium and a ventricle on each side of the heart. The natural mitral valve typically has two leaflets and the natural tricuspid valve typically has three. The aortic and pulmonary valves are referred to as "semilunar valves" because of the unique appearance of their leaflets, which are shaped somewhat like a half-moon and are often termed "cusps". The aortic and pulmonary valves typically each have three cusps.

Problems that can develop with heart valves are generally classified into two categories: (1) stenosis, in which a valve does not open properly and (2) insufficiency (also called regurgitation, in which a valve does not close properly. Stenosis insufficiency may occur concomitantly in the same valve or in different valves. Both of these abnormalities increase the workload placed on the heart. The severity of this increased workload on the heart and the patient, and the heart's ability to adapt to the increased workload, determine whether the abnormal valve will have to be surgically replaced (or, in some cases, repaired). US 5,344,442 describes a prosthetic cardiac valve.

A number of valve replacement options, including artificial mechanical valves and artificial tissue valves, are currently available. However, the currently available options have important shortcomings. Some of the available mechanical valves are durable, but tend to be thrombogenic and exhibit relatively poor hemodynamic properties. Some of the available artificial tissue valves may have relatively low thrombogenicity, but lack durability. Additionally, even artificial tissue valves often do not exhibit hemodynamic properties that approach the advantageous hemodynamic performance of a native valve.

### Summary of the Invention

Accordingly, there is a need in the art for an improved prosthetic heart valve that has advantageous hemodynamic performance, low thrombogenicity, and is durable.

In accordance with one aspect, the present invention comprises an atrioventricular replacement valve. A valve body has an inlet portion comprising an annulus and an outlet portion having at least two commissural attachment locations. The annulus has a periphery with scalloped edges.

In accordance with another aspect of the present invention, an atrioventricular replacement valve comprises a valve body having an inlet portion comprising an annulus and an outlet portion having at least two commissural attachment locations. The annulus has an annulus tilt angle in the range of about 5-20 degrees.

In accordance with still another aspect, the present invention comprises a replacement mitral valve. A valve body has an inlet and an outlet. The body includes an annulus at said inlet for attachment to a native tissue annulus. The body is comprised of an anterior leaflet and a posterior leaflet which meet along first and second hinge lines extending substantially from the annulus at the inlet towards the outlet. Each of the hinge lines at the annulus are disposed more than 60° and less than 90° from the midpoint of the anterior leaflet at the annulus.

In accordance with a further aspect of the present invention, an atrioventricular replacement valve comprises a valve body having a longitudinal axis. The body includes an inlet and an outlet, and is comprised of two leaflets which meet along first and second hinge lines extending substantially between the inlet and outlet. The first and second hinge lines at said inlet pass through a first plane which extends in a direction parallel to said longitudinal axis. The first and second hinge lines at said outlet pass through a second plane which extends in a direction parallel to said longitudinal axis. The first and second planes intersect at an angle.

In accordance with a still further aspect, a replacement atrioventricular valve comprises a tubular member having an inlet and an outlet. An anterior side of said member has a length between the inlet and outlet which is longer than that of a posterior side of said member.

In accordance with yet another aspect, the present invention provides a method of manufacturing a replacement atrioventricular valve. A sheet of tissue is provided. An anterior leaflet and a posterior leaflet are cut from said tissue. Cutting comprises cutting an inflow end of the anterior leaflet on a radius of curvature different than that of an inflow end of the posterior leaflet.

In accordance with an exemplary embodiment, a surgical method comprises providing a replacement atrioventricular valve having an inlet and an outlet. The valve comprises a tubular member having a longitudinal axis. A first direction along said axis extends from the inlet to the outlet. A second direction along said axis extends from the outlet to the inlet. The valve is comprised of a saddle-shaped annulus having an anterior saddle portion which extends further in said second direction than a posterior saddle portion of said annulus. The posterior saddle portion extends further in the second direction than intermediate saddle portions between the anterior and posterior saddle portions. The annulus is attached to a native tissue annulus with said anterior saddle portion abutting at least a portion of the fibrous trigon.

In accordance with a still further exemplary, aspect, an atrioventricular valve having a saddle-shaped annulus is provided. The atrioventricular valve is tested by placing said annulus in a seat having a shape complementary to the saddle-shaped annulus such that the annulus seals to the seat. The testing further comprises delivering a pulsating flow of fluid through the valve.

In accordance with another exemplary aspect, an atrioventricular replacement valve is provided. A valve body has an inlet, an outlet, an anterior leaflet and a posterior leaflet. The leaflets are connected to each other along hinge lines that extend from the inlet to the outlet. A first direction is defined generally from the inlet to the outlet along a longitudinal axis of the valve body, and a second direction is defined along the longitudinal axis generally opposite the first direction. The leaflets are scalloped at the outlet so that a distance in the second direction between the midpoints of each of the leaflets at the outlet and the hinge lines at the outlet is less than 4mm.

In accordance with a further aspect, an exemplary method of manufacturing a replacement heart valve is provided. A first leaflet and a second leaflet are provided, each leaflet comprising a distally-extending tab portion. A connector member is provided. The tab portion of the first leaflet is connected to the connector member. The tab portion of the second leaflet is also connected to the connector member.

For purposes of summarizing the invention and the advantages achieved over the prior art, certain aspects and advantages of the invention have been described herein above. Of course, it is to be understood that not necessarily all such aspects or advantages may be achieved in accordance with any particular embodiment of the invention. Thus, for example, those skilled in the art will recognize that the invention may be embodied or carried out in a manner that employs one or more aspects to achieve or optimize one advantage or group of advantages as taught herein without necessarily using other aspects or achieving other advantages as may be taught or suggested herein.

All of these aspects are intended to be within the scope of the invention herein disclosed. These and other aspects of the present invention will become readily apparent to those skilled in the art from the following detailed description of the preferred embodiments having reference to the attached figures, the invention not being limited to any particular preferred embodiments disclosed. The invention is disclosed as in the appended claims.

### Brief Description of the Drawings

Figure 1 is a cross-sectional view of a replacement human heart.
Figure 2 is a schematic perspective view of a heart valve having features in accordance with a preferred embodiment, shown in an open position.
Figure 3 is a schematic perspective view of the heart valve of Figure 2, shown in a closed position.
Figure 4A is a schematic pattern of a flat anterior leaflet portion used to form the heart valve of Figure 2.
Figure 4B is a schematic pattern of a flat posterior leaflet portion used to form the heart valve of Figure 2.
Figure 5 shows the leaflets of Figures 4A and B being sewn together according to an embodiment.
Figure 6 is a perspective view of a completed replacement heart valve constructed of the flat leaflets of Figures 4A and B.
Figure 7 schematically shows the heart valve of Figure 2 positioned in a left side of a patient's heart.
Figure 8 is a top end view of the perimeter edge shape of the annulus of the heart valve of Figure 2.
Figure 9 is a side view of the annulus of Figure 8 viewed along line 9-9 of Figure 8.
Figure 10 is an anterior side view of the annulus of Figure 8 viewed along line 10-10 of Figure 8.
Figure 11 is a schematic perspective view of another embodiment of a prosthetic mitral valve, showing a plane of the annulus of the valve.
Figure 12 is a schematic side view of the heart valve of Figure 11, illustrating an annulus tilt angle of the valve.
Figure 13 is a view of the heart valve of Figure 2 taken from an anterior side of the valve.
Figure 14 is a view of the heart valve of Figure 2 taken from a posterior side of the valve.
Figure 15 is a view of the heart valve of Figure 2 taken from a side between the anterior and posterior sides.
Figure 16 is an upstream end view of the valve of Figure 2.
Figure 17 is a downstream view of the heart valve of Figure 2.
Figure 18 is an upstream end view of a heart valve embodiment illustrating options for connecting the leaflets to one another.
Figure 19A is a downstream view of a heart valve embodiment as in Figure 18, shown in a closed position and having a first seam line configuration.
Figure 19B is a downstream end view of a heart valve embodiment as in Figure 18, shown in a closed position and having another seam line configuration.
Figure 20 is an upstream end view of another embodiment of a heart valve.
Figure 21 is a perspective view of a simulated annulus for use with a prosthetic valve test fixture.
Figure 22 is a side view of a prosthetic valve test fixture.
Figure 23 is a side view of the test fixture of Figure 22 with an embodiment of prosthetic valve mounted therein.
Figure 24 is a perspective view of the test fixture of Figure 22 arranged in another configuration and having an embodiment of a prosthetic valve mounted therein.
Figure 25 is a side view of the arrangement of Figure 24.
Figure 26A is a schematic pattern of a flat anterior leaflet portion used to form an embodiment of a replacement heart valve.
Figure 26B is a schematic pattern of a flat posterior leaflet portion used to form an embodiment of a replacement heart valve.
Figure 26C is a schematic pattern of a connecting portion adapted to hold tab portions of the anterior and posterior leaflets of Figures 26A-B together.
Figure 26D is a schematic pattern of a flat anterior leaflet portion used to form a two-piece embodiment of a replacement heart valve.
Figure 26E is a schematic pattern of a flat posterior leaflet portion used to form a two-piece embodiment of replacement heart valve.
Figure 27 is a perspective view of Step 6.1, the formation of the first seam line, in the assembly of a replacement valve.
Figure 28 is a perspective view of Step 6.2, the completion of the first and second seam lines, in the assembly of a replacement valve.
Figure 29 is a perspective view of Step 6.4, the formation of the slotted tab, in the assembly of a replacement valve.
Figure 30 is a perspective view of Step 6.5, the formation of the slotted tab, in the assembly of a replacement valve.
Figure 31 is a perspective view of Step 6.6, the formation of the slotted tab, in the assembly of a replacement valve.
Figure 32 is a schematic chart of the sewing locations of Step 6.7 , the formation of the slotted tab, in the assembly of a replacement valve.
Figure 33 is a perspective view of Step 6.8, the finished slotted table, in the assembly of a replacement valve.
Figure 34 is a top view of Step 6.9, the cloth strip, in the assembly of a replacement valve.
Figure 35 is a perspective view of Step 6.10, the alignment of the cloth tab along the surface of the tissue tab, in the assembly of a replacement valve.
Figure 36 is a perspective view of Step 6.11, the folding of the cloth tab over the end of the tissue tab, in the assembly of a replacement valve.
Figure 37 is a schematic chart of the sewing locations of Step 6.12, the formation of the tab, in the assembly of a replacement valve.
Figure 38 is a perspective view of Step 6.13, the formation of the tab, in the assembly of a replacement valve.
Figure 39 is a perspective view of Step 6.14, the sewing of the ends of sewing ring, in the assembly of a replacement valve.
Figure 40 is a perspective view of Step 6.15, the wrapping and aligning of the sewing ring with the seam lines, in the assembly of a replacement valve.
Figure 41 is a perspective view of Step 6.16, the making of marker stitches on the sewing ring, in the assembly of a replacement valve.
Figure 42 is a perspective view of Step 6.17, showing the anterior marking stitches, in the assembly of a replacement valve.
Figure 43 is a perspective view of Step 6.18, showing the posterior marking stitch, in the assembly of a replacement valve.
Figure 44 is a perspective view of Step 6.19, the assembled apparatus and the storage of the assembled apparatus, in the assembly of a replacement valve.

### Detailed Description of the Preferred Embodiment

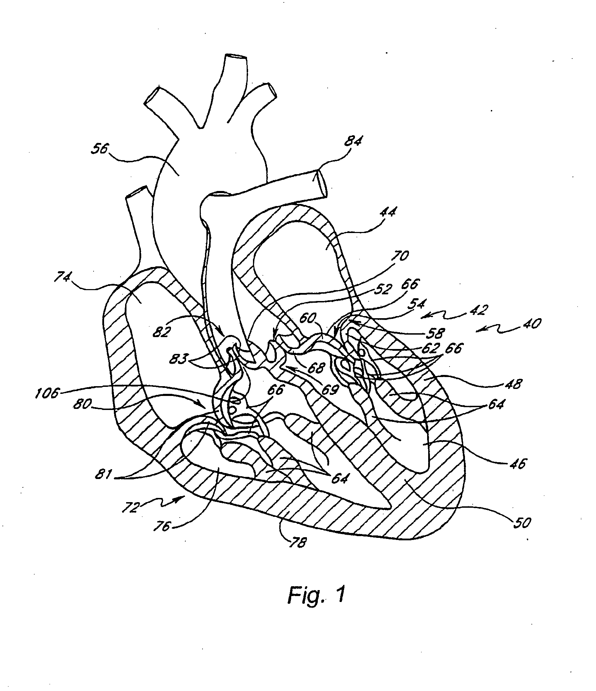

Figure 1 is a cross-sectional cutaway depiction of a typical human heart 40. The left side 42 of the heart 40 includes a left atrium 44 and a left ventricular chamber 46. The left ventricle 46 is defined between a left ventricular wall 48, a septum 50, an aortic valve assembly 52 and a mitral valve assembly 54. The mitral valve assembly 54 is positioned generally between the left ventricle 46 and the left atrium 44 and regulates blood flow from the atrium 44 into the ventricle 46. The aortic valve assembly 52 is positioned atop the left ventricle 46 and regulates blood flow from the left ventricle 46 into an aorta 56.

The mitral valve assembly 54 includes a mitral valve annulus 58; an anterior leaflet 60 (sometimes called the aortic leaflet, since it is adjacent to the aorta); a posterior leaflet 62; two papillary muscles 64, which are attached at their bases to the interior surface of the left ventricular wall 48; and multiple chordae tendineae 66, which extend between the mitral valve leaflets 60, 62 and the papillary muscles 64. Generally, numerous chordae 66 connect the leaflets 60, 62 and the papillary muscles 64, and chordae from each papillary muscle 64 are attached to both of the valve leaflets 60 and 62.

The aorta 56 extends generally upwardly from the left ventricular chamber 46, and the aortic valve 52 is disposed within the aorta 56 adjacent the left ventricle 46. The aortic valve 52 comprises three leaflets or cusps 68 extending from an annulus 69. Portions of each cusp 68 are attached to the aortic wall 70 at commissural points (not shown) in a known manner.

The right side 72 of the heart 40 includes a right atrium 74 and a right ventricular chamber 76. The right ventricle 76 is defined between a right ventricular wall 78, the septum 50, a tricuspid valve assembly 80 and a pulmonary valve assembly 82. The tricuspid valve assembly 80 is positioned generally between the right atrium 74 and the right ventricle 76 and regulates blood flow from the right atrium 74 into the right ventricle 76. A plurality of tricuspid valve leaflets 81 are connected by chordae tendineae 66 to papillary muscles 64. The pulmonary valve assembly 82 is disposed within a pulmonary artery 84, which leads from the right ventricle 76 to the lungs. The pulmonary valve assembly 82 has a plurality of cusps 83, and regulates blood flow from the right ventricle 76 into the pulmonary artery 84.

The native mitral and tricuspid valve leaflets 60, 62, 81, as well as the aortic and pulmonary valve cusps 68, 83, are all passive structures in that they do not themselves expend any energy and do not perform any active contractile function. Instead, they open and close in response to differential pressures of blood on either side of the valve.

As discussed above, it is sometimes necessary to replace a native heart valve with a prosthetic valve. The native valve can be removed by cutting at or about the valve annulus. In semilunar valves, the valve's commissural attachment points are also cut out. In atrioventricular valves, the corresponding papillary muscles and/or chordae tendineae are cut. Once the native valve is removed, a replacement valve is installed. Sutures or other attachment methods are used to secure an inflow annulus of the replacement valve to the valve annulus 58 vacated by the native valve. Downstream portions of the replacement valve preferably are attached to commissural attachment points, papillary muscles and/or chordae tendineae, as described below.

A number of embodiments of prosthetic heart valves are described below. These embodiments illustrate and describe various aspects of the present invention in the context of a replacement mitral valve. Although the valve embodiments discussed and presented below are prosthetic mitral valves, it is to be understood that aspects of these embodiments can be applied to other types of heart valves.

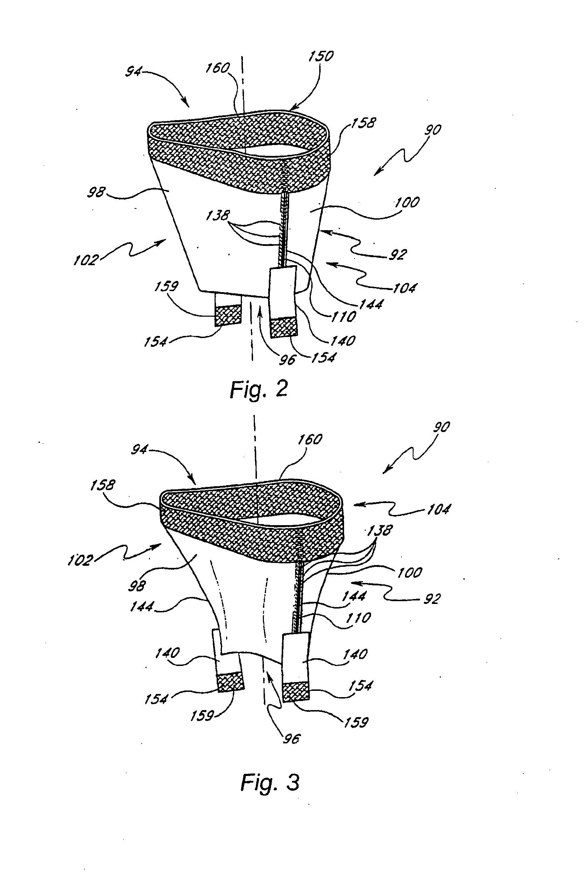

Figures 2 and 3 show an embodiment of a replacement mitral valve 90 in an open and closed position, respectively. The valve 90 comprises a valve body 92 having an inlet portion 94 and an outlet portion 96. The valve body 92 comprises an anterior leaflet 98 and a posterior leaflet 100 that are disposed generally on anterior and posterior sides 102, 104, respectively, of the valve 90. The leaflets 98, 100 preferably are formed of a thin, flexible material, and are attached to one another along seam lines 110 so as to form a generally tubular valve 90 having a longitudinal center axis L_{c}.

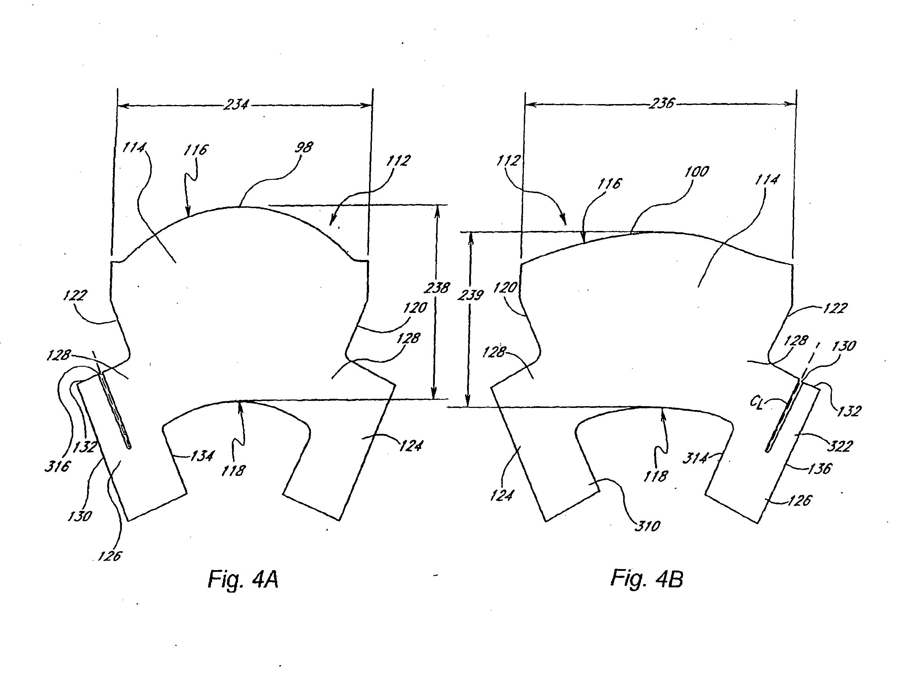

To construct the valve embodiment depicted in Figures 2 and 3, the anterior and posterior leaflets 98, 100 preferably are cut out of a thin, flat and flexible material according to specialized patterns such as the patterns 112 depicted in Figures 4A and B.

After the flat, flexible leaflets 98, 100 have been cut out, they are sewn together in order to form the valve. US 2002/0052651 entitled PROSTHETIC HEART VALVE, discusses cutting leaflets out of a thin, flat, and flexible material according to a pattern or template and then sewing the leaflets together to make a heart valve. The entire disclosure of this application is hereby incorporated by reference.

With continued reference to Figures 4A and B, each flat leaflet 98, 100 has a main body 114 having an inflow end 116, an outflow end 118 and first and second side edges 120, 122 extending therebetween. The leaflets 98, 100 are scalloped on both their inflow and outflow ends 116, 118. First and second distal tab portions 124, 126 extend outwardly from the respective side edges 120, 122 of each leaflet body 114 and extend longitudinally downstream of the outflow end 118 of each leaflet 98, 100.

As shown in the figures, the shape of the scalloped inflow end 116 of the anterior leaflet 98 is different than the shape of the scalloped inflow end 116 of the posterior leaflet 100. More specifically, the inflow end of the anterior leaflet has a radius of curvature that is different than the radius of curvature of the inflow end of the posterior leaflet. Similarly, the radius of curvature of the outflow end 118 of the anterior leaflet 98 is different than the radius of curvature of the outflow end 118 of the posterior leaflet. 100.

The scallop of the outflow ends is not as pronounced as that of the inflow ends. Preferably, a distance from the downstream-most portion to the upstream-most portion of each outflow end is less than about 4mm. More preferably, the distance is between about 1-3mm, and most preferably is about 1 mm..

Each of the tabs 124, 126 communicates with the leaflet main body 114 through a neck portion 128. An elongate slot 130 is formed in the second tab 126. The slot 130 extends distally from a proximal edge 132 of the tab 126 to a point just distal of the distal edge 118 of the leaflet main body 114. A longitudinal center line C_{L} of the slot 130 preferably is positioned about 2/3 of the way from an inner edge 134 of the tab 126 to an outer edge 136 of the tab 126.

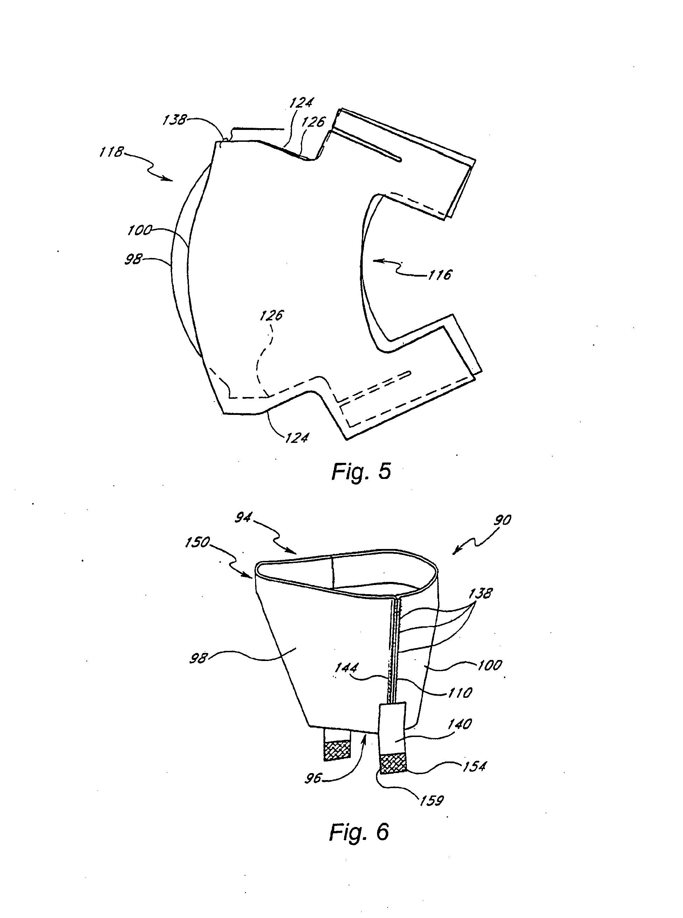

With reference also to Figures 5 and 6, the valve 90 is constructed by aligning the first side edge 120 of one leaflet with the second side edge 122 of another leaflet so that the inner surfaces of the aligned leaflets are facing one another. The side edges 120, 122 are then sutured together starting at the inflow ends 118 and progressing toward the outflow ends 116. The stitches extend along a substantially straight scam line 110 adjacent the leaflet edges, and preferably include locking knots 138, which allow the integrity of the entire seam to be preserved even if a portion of the scam is cut or broken. The stitches 138 preferably are spaced approximately 1 mm from the edges and are spaced 1 to 1-1/2 mm apart.

The first and second distal tabs 124, 126 of adjacent leaflets are folded over one another as discussed in the above-referenced application PROSTHETIC HEART VALVE so as to form longitudinally extending portions 140. In this manner, adjacent leaflets 98, 100 are securely attached to one another and the longitudinally extending portions 140 extend downstream from the main body 114 of the leaflets 98, 100.

In the illustrated embodiments, sutures of the scam lines 110 do not extend into the distal-most portion of the leaflets. Instead, the longitudinally extending portions 140 generally hold the leaflets 98, 100 together at their distal ends. This reduces the stress concentrations and possible friction and wear associated with sutures placed at the outflow ends of leaflets, where folding of the leaflets during repeated opening and closing of the valves is most pronounced.

Suturing the inner surfaces of the leaflets together along the seam lines 110 provides a slight biasing of the leaflets 98, 100 toward each other to aid in closing the valve 90 without significantly restricting blood flow through the valve 90 when the valve is open. In the illustrated embodiment, each seam 110 functions as a hinge line 144 about which the leaflets preferentially bend when opening and closing. It is to be understood that any type or method of attaching the leaflets can be used. In additional embodiments, a tubular material can be used for the valve body. Such a tubular body may or may not include seams. Preferably, however, the tubular body will have hinge lines defining adjacent leaflets.

The term "hinge line" is used broadly in this specification to refer to a portion of a valve at which adjacent leaflets meet and/or to a portion of the valve which preferentially bends during valve opening and closure. For example, in several embodiments discussed below, leaflets are connected along at least a portion of a seam line. Such a seam line is also appropriately considered a hinge line. In embodiments wherein adjacent leaflets are constructed of a continuous piece of material, there may be no seam line between the leaflets, yet the leaflets still meet at a hinge line at which the leaflets bend relative to one another.

The flat, flexible leaflet portions 98, 100 depicted in Figures 4A and B are sewn together to help form the prosthetic valve 90 shown in Figures 2, 3 and 6. The shapes of the leaflet patterns 112 and the manner of sewing the leaflets together determines certain aspects of the valve, such as the shapes of an inflow annulus 150 and the outflow portion 96, and the disposition of the hinge lines 144. It is to be understood that other valves having other aspects, such as having annulus shapes and hinge line dispositions that differ from those discussed herein and illustrated in the drawings, can be constructed by employing leaflets having different patterns and/or connecting the leaflets according to other methods. For instance, the illustrated heart valve body 90 has a generally frustoconical shape; other shapes, such as cylindrical, can also be used for an embodiment of a heart valve.

With reference again to Figures 2 and 3, the inflow annulus 150 of the valve body 92 is scalloped so that it is generally saddle-shaped about its perimeter. This annulus shape helps the valve 90 to fit securely in an annulus vacated by a native mitral heart valve, and will be discussed in more detail below. The longitudinally extending portions 140 of the valve 90 extend downstream beyond the outflow annulus 96 of the valve. Commissural attachment locations 154 are defined on each of these longitudinally extending portions 140. As such, these portions are termed "commissural tabs." The commissural tabs 140 extend generally along the hinge lines 144. The valve body 92 generally folds about the hinge lines 144 during valve closure (see Figure 3) so that the valve leaflets 98, 100 coapt, thus closing the valve 90.

In the illustrated embodiment, the leaflets are formed from thin and flexible equine pericardium. However, it is to be understood that several types of materials, whether biological or synthetic, can be used to form the leaflets. For example, bovine, porcine, and kangaroo pericardial tissue may appropriately be used. Synthetic materials such as polyesters, Teflon, woven or knitted cloth, etc., can also be used. Materials can be selected using a general guideline that the more pliable, thin and strong the material is, the better. Additionally, it is advantageous for the material to be as nonthrombogenic as possible.

In a preferred embodiment, a non-contact cutter, such as a carbon dioxide laser, is used to cut individual leaflets out of flat sheets of material. As discussed above, the material may be animal tissue or a synthetic material. Varying certain laser parameters, such as pulse power, cutting speed, and pulses per inch enables an operator to choose a number of arrangements that will provide appropriate cutting and fusing of the materials. Further details regarding cutting leaflets is provided in copending application "Method of Cutting Material For Use In Implantable Medical Device", U.S. Serial Number 10/207,438, filed July 26, 2002.

In a preferred embodiment, a plotted laser cutter, such as an M-series laser available from Universal Laser Systems of Scottsdale, Arizona, is used to precisely cut leaflets out of flat layers of the material. The plotter preferably is controlled by a computer in order to provide precision and repeatability.

Other cutting media and methods may be used to obtain repeatable, precise cutting of leaflets. Such cutting media can include a razor, die-cutter, or a jet of fluid and/or particles. The cutting methods used should reduce fraying of cloth materials and avoid delamination of tissue.

The flexible leaflets 98, 100 are readily movable between the open valve position shown in Figure 2 and the closed position shown in Figure 3. When the blood pressure in the heart upstream of the valve 90 is greater than it is downstream of the valve, the blood will push against the flexible leaflets 98, 100, which will bend about their hinge lines 144 to the open position shown in Figure 2, thus enabling blood to flow through the valve 90. However, when the blood pressure is greater downstream of the valve, the pressure will bend the leaflets inwardly and force them into engagement with one another as shown in Figure 3. When the leaflets 98, 100 are engaged (coapted), the valve is closed and blood generally is prevented from passing through the valve 90.

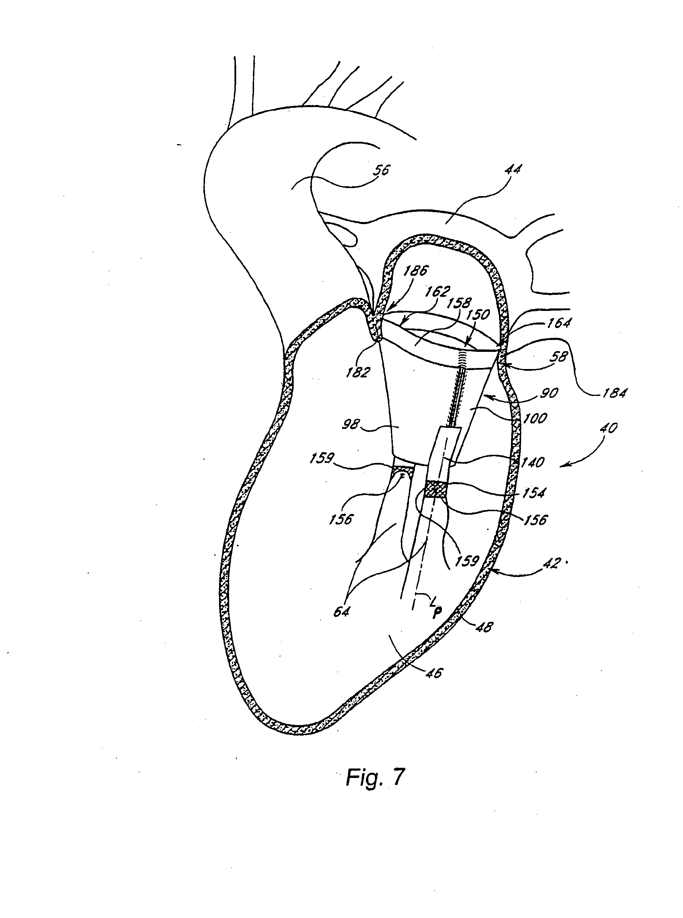

Figure 7 schematically shows the replacement mitral valve 90 of Figure 2 installed in the left side 42 of a heart 40. The inflow annulus 150 of the valve 90 is sutured into the annulus 58 vacated by the native mitral valve, and the valve leaflets 98, 100 extend generally downwardly into the left ventricle 46. The valve 90 opens generally downwardly into the ventricle 46 so as to allow blood to flow from the left atrium 44 into the left ventricle 46. In the illustrated embodiment, sutures 156 connect the commissural tabs 140 to the papillary muscles 64, which extend from the ventricle wall 48. In an additional embodiment, the commissural tabs can be at least partly connected to chordae tendineae that extend from the papillary muscles. Attaching the commissural tabs to papillary muscles or chordae tendineae helps to hold the valve in a closed position and to prevent the valve leaflets from prolapsing during systole, when blood pressure in the ventricle is comparatively high.

The inflow annulus 150 sustains significant forces during the repeated opening and closing of the valve 90 and during the pulsed flow of blood through the valve. In the embodiment illustrated in Figures 2, 3 and 7, an annular sewing cuff 158 is provided at the inflow annulus 150 to reinforce the inflow annulus. The sewing cuff 158 preferably comprises a woven or knit cloth material, such as a polyester material, that is sutured or otherwise attached to the inflow annulus 150. In a preferred embodiment, the sewing cuff comprises polyethylene tereplithalate that has been knitted in a velour fashion.

When the valve 90 is installed, the cloth facilitates growth of fibrous body tissue into and around the sewing cuff 158. This fibrous ingrowth further secures the cuff 158 and valve 90 to the heart annulus, and better establishes a seal between the valve's inflow annulus 150 and the native annulus. Additionally, as tissue grows into and around the woven material, natural cells are deposited between the blood flow and the material. Thus, tissue ingrowth effectively isolates the synthetic cloth material from the blood flow and, consequently, reduces thrombogenicity.

In addition to cloth reinforcement, the leaflet material can also be folded over a short distance and stitched into place at the inflow annulus 150 for increased reinforcement. Preferably, the material is folded over itself a distance of about 1-5mm and more preferably about 2-3mm. Folding the leaflet material over itself at the inflow annulus strengthens the annulus and provides a reinforcement layer to strengthen the connection between the inflow annulus 150 and the native mitral valve annulus.

In the illustrated embodiment, the cloth reinforcement comprises a flexible but generally non-elastic material. When the prosthetic valve 90 is sewn into place, the sewing cuff 158 is sewn to the heart's mitral annulus 58. The sewing cuff 158 is flexible and generally will change shape along with the annulus. However, the cuff is also generally nonelastic and will constrain the mitral annulus from expanding beyond the size of the cuff. Thus, the perimeter of the mitral annulus will not become greater than the perimeter of the sewing cuff 158. As such, the prosthetic valve 90 can be especially helpful in treating certain diseased hearts. For example, if a heart is experiencing congestive failure (CHF), certain portions of the heart, including one or more valve annulus, may enlarge. When performing surgery on such a heart, a clinician can install a prosthetic mitral valve 90 having an annulus perimeter that is smaller than the enlarged annulus perimeter of the diseased heart. Due to the above-discussed properties of the sewing cuff 158, the prosthetic valve 90 will reduce and limit the size of the diseased heart's mitral annulus 58 to the size of the sewing cuff 158.

With continued reference to Figures 2, 3 and 7, the downstream portions 154 of the commissural tabs 140 are covered with a reinforcement portion 159 which preferably comprises a woven or knit cloth material made of biocompatible material such as polyester. In a preferred embodiment, the material comprises polyethylene terephthalate as is used in the annular sewing cuff 158. This material is sutured or otherwise attached to each commissural tab 140 at the commissural attachment locations 154 of the tab. As with the annular sewing cuff 158 discussed above, each reinforcement cloth portion 159 provides reinforcement to the corresponding commissural tab 140 at the commissural attachment location 154 and also facilitates growth of fibrous body tissue into and around the cloth material. As such, fibrous tissue will grow into and cover the woven material, and the cloth is generally isolated from direct contact with blood flow.

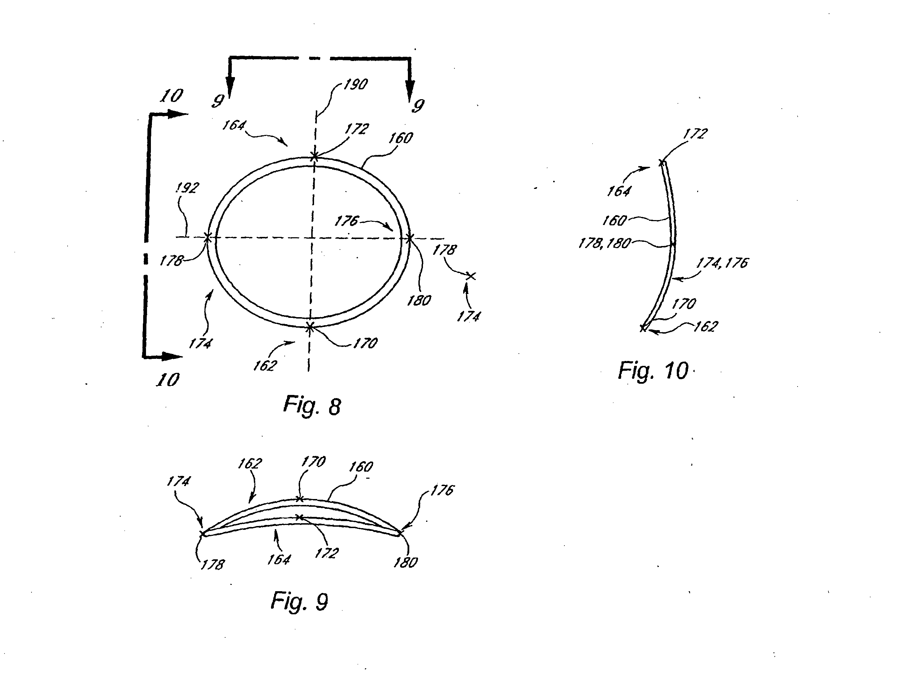

As discussed above and as shown in Figures 2, 3 and 7, the inflow annulus 150 preferably is scalloped so as to have a generally saddle-like shape. In order to aid discussion of the annulus shape, Figures 8-10 depict various views of only a peripheral edge 160 of the saddle-shaped inflow annulus 150. As shown, the annulus peripheral edge 160 has relatively high anterior and posterior portions 162, 164. An anterior high point 170 is disposed generally centrally in the anterior high portion 162 and a posterior high point 172 is disposed generally centrally in the posterior high portion 164. As best shown in Figures 9 and 10, the anterior high point 170 generally is higher than the posterior high point 172. The anterior and posterior relatively high portions 162, 164 are separated by first and second relatively low portions 174, 176, which include first and second low points 178, 180, respectively.

With reference also to Figure 7, the anterior high portion 162 of the annulus 150 is configured to fit in an anterior portion 182 of a heart's mitral annulus and the posterior high portion 164 is configured to fit in a posterior portion 184 of a heart's mitral annulus so that the anterior portion 162 is generally vertically higher than the posterior high portion 164. In this configuration, the anterior portion 162 is positioned generally adjacent a fibrous trigon region 186 of the heart.

With specific reference to Figure 8, the annulus peripheral edge 160 preferably has a non-circular shape, such as an ovoid, oval or elliptical shape, when viewed from above. In the illustrated embodiment, the anterior and posterior high points 170, 172 are oriented generally 180° from one another along the periphery 160 of the annulus 150. Similarly, the first and second low points 178, 180 are oriented generally 180° from one another. A diameter of the annulus taken across the high points 170, 172 is less than a diameter taken across the low points 178, 180.

Figure 8 shows a first axis 190 extending between the anterior and posterior high points 170, 172, and a second axis 192 extending between the first and second low points 178, 180. The annulus edge 160 is generally symmetric about the second axis 192, but is slightly asymmetric about the first axis 190. It is to be understood that, in additional embodiments, the annulus can be symmetric about both axes, one or the other axis, or can be asymmetric about both axes. It is also to be understood that, in additional embodiments, and in other types of replacement valves, the respective high points and low points may have different angular relations relative to one another. For example, in one additional embodiment, the low points each are less than 90° from the anterior high point, thus the minimum angular distance between the low points is less than 180°.

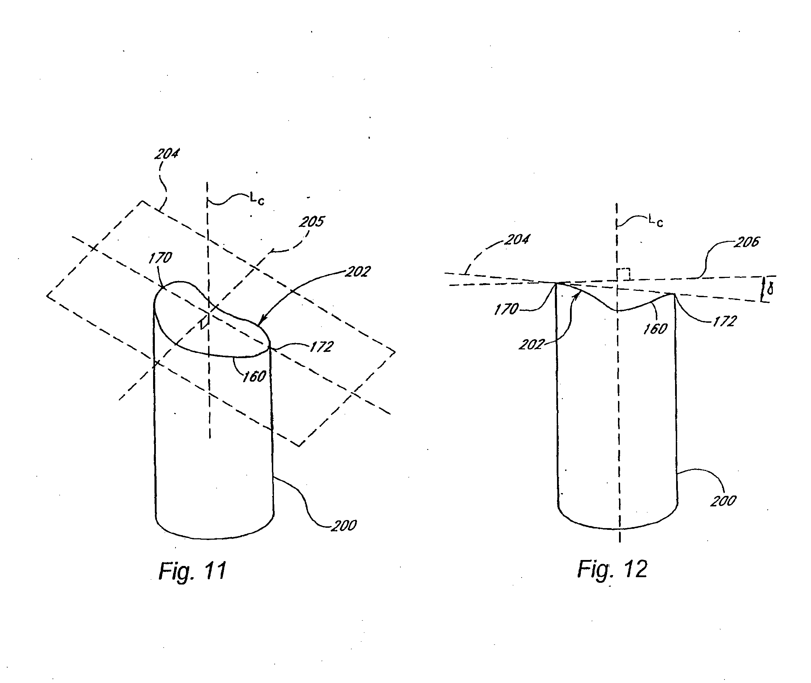

With reference next to Figures 11 and 12, another embodiment of a replacement mitral valve 200 is illustrated. The valve 200 is generally cylindrical and has an inflow annulus 202 having a saddle-like shape such as the inflow annulus 150 shown in Figures 8-10. The valve 200 is shown in an open position and has a longitudinal center line L_{c} extending therethrough. Figures 11 and 12 show a plane of the annulus 204 of the valve. The term "plane of the annulus" 204, as used herein, refers to an imaginary plane 204 that (a) touches the valve annulus 202 at least two spaced locations along the periphery 160 of the valve annulus 202; (b) is disposed so that no portion of the valve penetrates the plane 204; and (c) is oriented so that an imaginary line 205 perpendicular to the longitudinal axis L_{c} of the valve is contained within the plane 204. In the illustrated embodiment, the plane of the annulus 204 touches the valve annulus 202 at the anterior and posterior high points 170,172.

The plane of the annulus 204 helps define the disposition of the annulus 202 relative to the rest of the valve 200. With specific reference to Figure 12, an annulus tilt angle γ of the annulus is illustrated. The term "annulus tilt angle" γ is defined herein as the minimum angle between a plane 206 perpendicular to the longitudinal axis L_{c} of the valve 200 and the plane of the annulus 204. In the preferred embodiment, the annulus tilt angle γ is in the range of about 5° to 25°, and more preferably is about 12° to 20°.

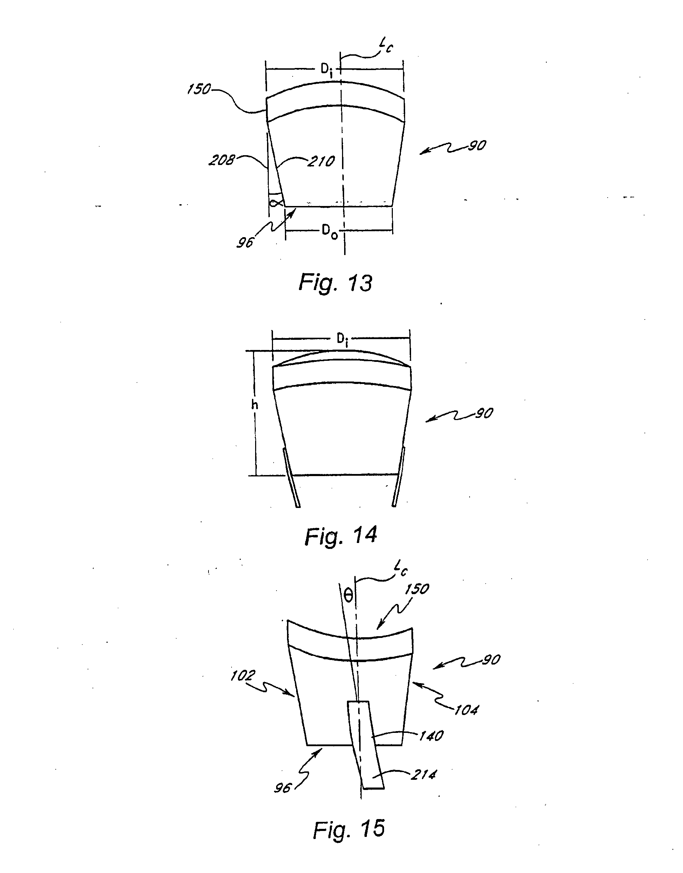

With next reference to Figures 13-15, and with specific reference to Figure 13, the illustrated replacement valve embodiment 90 has a generally tapered shape. That is, the maximum diameter Dₒ at the outflow annulus 96 is less than the maximum diameter Dᵢ of the valve at the inflow annulus 150. The taper of the valve preferably is such that the outflow diameter Dₒ is about 0%-10% smaller than the overall inflow diameter Dᵢ. More preferably, the outflow diameter is about 5% smaller than the overall inflow diameter Dᵢ. The valve taper can also be expressed in terms of a draft angle α of the valve. The draft angle α is the angle between a line 208 parallel to the longitudinal axis L_{c} of the valve 90 and a line 210 extending from a point on the inflow annulus 150 to a point on the outflow annulus 96, wherein the lines 208, 210 are coplanar. Preferably, the valve draft angle is greater than 0° but less than about 60°.

With specific reference next to Figure 14 & 15, the relationship between the inflow diameter Dᵢ and a length h of the valve 90 affects closure and hemodynamic properties of the valve. The length h of the leaflets preferably is between about 50%-100% of the maximum inflow diameter Dᵢ of the valve, and more preferably is between about 75%-90% of Dᵢ. In the illustrated embodiment, the maximum inflow diameter Dᵢ is between about 20-45mm, and more preferably is between about 25-32mm; the maximum length h of the leaflets preferably is between about 15-30mm, and more preferably is between about 20-26mm.

With next reference to Figure 15, an embodiment of a heart valve 90 is illustrated wherein the commissural tabs 140 downstream of the outflow annulus 96 of the valve 90 are angled relative to the longitudinal axis L_{c} of the valve. More specifically, the commissural tabs 140 are angled toward the posterior side 104 of the valve 90. As such, downstream ends 214 of the commissural tabs 140 are positioned closer to the ventricle wall and thus closer to the papillary muscles so as to aid attachment of the commissural tabs 140 to the papillary muscles and/or chordae tendineae. In Figure 15, the downstream ends 214 of the commissural tabs are disposed at an angle θ relative to the longitudinal axis L_{c} of the valve.

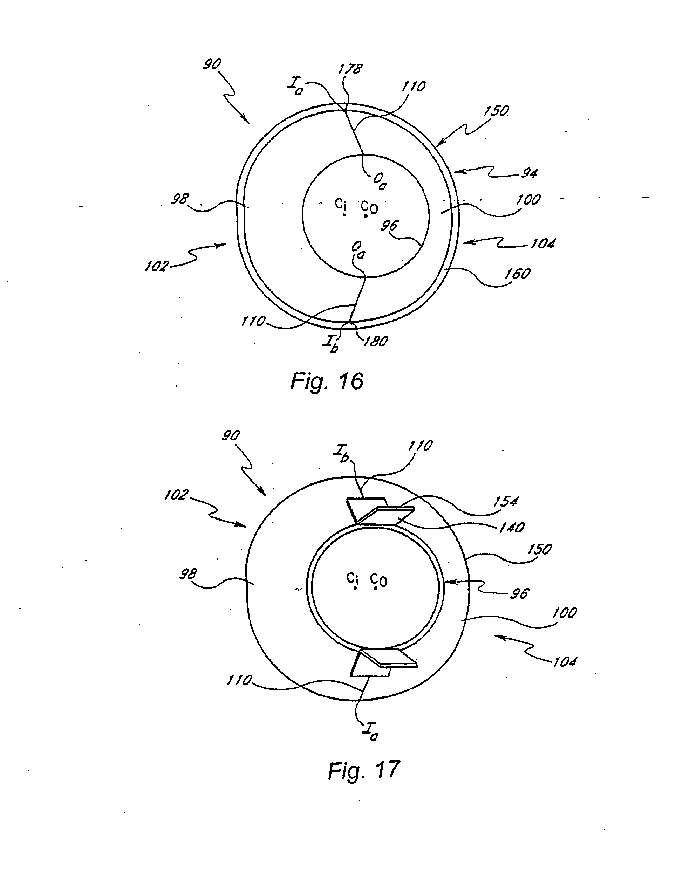

Figures 16 and 17 depict schematic views of the valve 90 of Figure 2 viewed from points upstream and downstream of the valve. As shown, the anterior and posterior leaflets 98, 100 preferably are sewn to one another along at least a portion of the seam lines 110 in a manner so that the seam lines 110 are slanted generally toward the posterior side 104 of the valve as the seam 110 extends from the inflow end 94 to the outflow end 96 of the valve 90. As such, and as shown in Figures 16 and 17, a center Cₒ of the outflow annulus 96 is offset in the posterior direction from a center Cᵢ of the inflow annulus 150. In the illustrated embodiment, the commissural tabs 140 are generally aligned with the seam lines 110; thus, this arrangement directs the commissural tabs 140 toward the ventricular wall and places the commissural attachment positions 154 generally close to the ventricular wall.

In the embodiment depicted in Figure 16, the upstream ends Iₐ, I_{b}, of each seam 110 are disposed generally 180° from one another, and are generally aligned with the first and second low points 178, 180 of the annulus 150 (see Figures 8-10). The downstream ends Oₐ, O_{b} of each seam line 110 are also disposed generally 180° from one another.

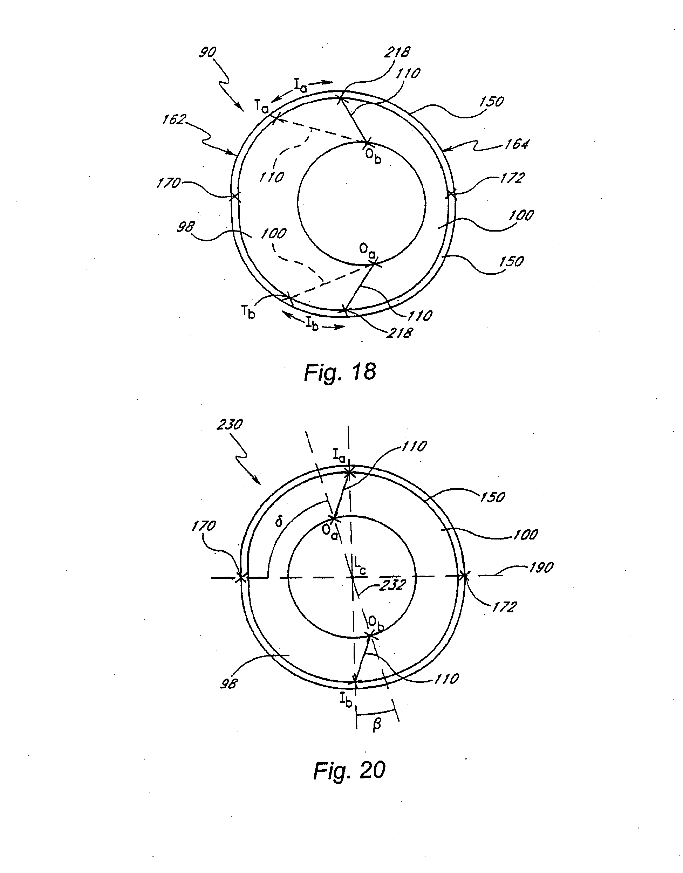

With next reference to Figure 18, in other embodiments the position of the upstream ends Iₐ, I_{b} of the seams 110 can be varied to tailor the performance of the valve 90. As discussed above and shown in Figure 7, the valve annulus 150 preferably is placed in the heart's mitral annulus 58 so that at least part of the anterior portion 162 of the valve annulus 150 is disposed adjacent the fibrous trigon 186 of the heart 40. In Figure 18, the points Tₐ and T_{b} schematically indicate the limits of the portion of the annulus 150 attached to the fibrous trigon 186. Thus, the annulus 150 is attached to the fibrous trigon 186 between points Tₐ and T_{b}. Preferably, the upstream ends Iₐ, I_{b} of the seam lines are positioned anywhere between the fibrous trigon connection limits Tₐ and T_{b} and a pair of locations 218 on the annulus that are about 180° from one another. As such, the anterior leaflet 98 subtends less than or about 180° of the inflow annulus 150.

A midpoint of the annulus in the anterior leaflet bisects the anterior leaflet along the inflow annulus. In the illustrated embodiment, the anterior high point 170 of the valve is the midpoint of the annulus in the anterior leaflet. In one embodiment, the valve is adapted to be installed so that the midpoint is arranged generally centrally in the fibrous trigon 186 portion of the native annulus 58. In this embodiment, the upstream ends Iₐ, I_{b} of the seam lines are disposed less than about 90° from the midpoint. Preferably, the upstream ends Iₐ, I_{b} of the seam lines are each disposed more than about 60° from the midpoint. More preferably, the upstream ends Iₐ, I_{b} of the seam lines are each disposed between about 60-85, and still more preferably between about 70-80°, from the midpoint.

With continued reference to Figure 18, a midpoint also bisects the posterior leaflet along the inflow annulus. In the illustrated embodiment, the posterior high point 172 is the midpoint of the posterior leaflet. The downstream ends Oₐ, O_{b} of the seam lines 110 are each disposed less than 90° from the midpoint 172. Most preferably, the downstream ends Oₐ, O_{b} are disposed between about 80-89° from the midpoint. In another embodiment, the downstream ends Oₐ, O_{b} are disposed less than 180° from one another.

It is to be understood that, in additional embodiments, the downstream ends of the seam lines can vary over a wide range of angles relative to one another as desired to enhance valve closure and hemodynamic properties. In at least some of the above-described embodiments, commissural tabs extend downstream from the seam lines and comprise commissural attachment locations. Thus, the positioning of the commissural attachment locations can be determined at least in part by the arrangement of the downstream ends Oₐ, O_{b} of the seam lines 110.

Further, and with reference also to Figure 7, the commissural tabs 140 preferably are attached to the papillary muscles 64 so that the downstream ends Oₐ, O_{b} of the seam lines 110 are generally aligned with an axis Lₚ of the corresponding papillary muscle 64. The papillary axis Lₚ is the general direction in which the corresponding papillary muscle expands and contracts during use.

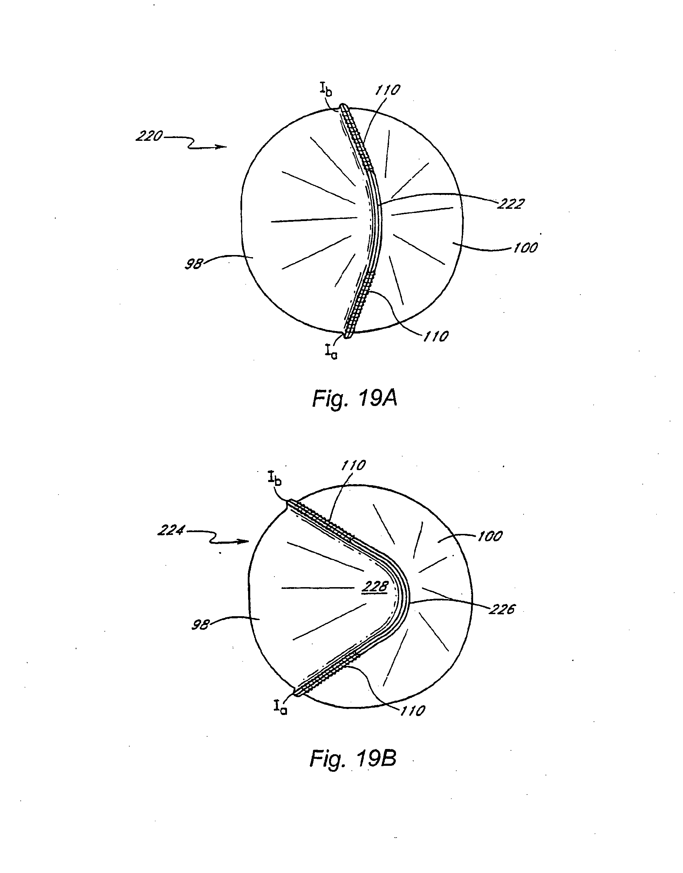

The arrangement of the seam lines, as well as the size and shape of the anterior and posterior leaflets, helps determine the hemodynamic attributes of the valve and the valve's behavior during closure. Thus, valve embodiments having different seam line arrangements and/or leaflet shapes can be expected to exhibit different hemodynamic attributes and closure behavior. For example, Figure 19A shows a downstream view of a valve 220 wherein the upstream ends Iₐ, I_{b} of the seam lines 110 are about 180° relative to one another and the downstream ends Oₐ, O_{b} of the seam lines 110 are also about 180° relative to one another. In this embodiment, the anterior and posterior leaflets 98, 100 coapt during closure along a mildly curved coaptation line 222. With next reference to Figure 19B, an embodiment of a replacement valve 224 is presented wherein the posterior leaflet 100 is much wider than the anterior leaflet 98 and the angle between opposing upstream ends Iₐ, I_{b} is less than about 180° so that the anterior leaflet 98 subtends the posterior leaflet 100. In this embodiment, the coaptation line 226 of the leaflets at closure is also curved, yet more dramatically than in the embodiment in which the anterior and posterior leaflets 98, 100 are close to or generally the same size. In both of the illustrated embodiments, the seam lines 110 are arranged such that the coaptation line 222, 226 of the valve leaflets 98, 100 generally resembles a smile, as is the case with a natural mitral valve. Preferably, the valve 224 is arranged so that the anterior leaflet 98, when closed, defines a trough 228 along which blood can flow. The trough 228 defines a passageway from the ventricle to the aorta and improves the hemodynamic properties of the valve.

The above discussion illustrates that several embodiments of valves can be constructed over a range of seam line configurations and having a corresponding range of leaflet shapes and sizes. As shown, the seam lines do not necessarily extend in the same direction as the flow of blood through the valve. More specifically, in some embodiments, a plane through the upstream ends Iₐ, I_{b} of the seam lines 110 and at least one commissural attachment location intersects a longitudinal center line L_{c} of the valve. Such a construction can assist in the creation of a suitable trough 228 during valve closure.

Figure 20 schematically illustrates another embodiment of a replacement mitral valve 230 that compensates for twisting of the ventricle during systole. When the valve 230 is at rest, the downstream ends of the seam lines are generally twisted about the longitudinal axis L_{c} relative to the upstream ends. This helps to improve closure of the valve and lessens creasing of the valve during operation.

In the illustrated embodiment, the valve 230 is depicted so that the longitudinal axis L_{c} extends straight into the page. The upstream ends Iₐ, I_{b} of the seam lines 110 lie in a plane that is parallel to the longitudinal axis. The downstream ends Oₐ, Oₐ of the seam lines lie in another plane that is parallel to the longitudinal axis. The upstream plane intersects the downstream plane at an angle β. Preferably, the angle β is between about 2-30° and, more preferably, is between about 3-10°. Most preferably, the angle β is between about 5-6°.

Twist of the downstream ends of the seam lines can also be measured relative to the anterior and posterior high points 170, 172 of the valve, without being tied to the position of the upstream ends Iₐ, I_{b}. In the embodiment shown in Figure 20, the anterior and posterior high points 170, 172 are disposed generally 180° relative to one another and a high point plane extends through the high points 170, 172 and parallel to the longitudinal axis L_{c}. The downstream ends Oₐ, O_{b} of the seam lines 110 are disposed on opposite sides of the high point plane, but are not disposed symmetrically about the high point plane. Instead, an angular offset δ is defined as the angle δ between the high point plane and a plane 232 extending through the downstream ends Oₐ, O_{b} and parallel to the longitudinal axis. In the illustrated embodiment, the angular offset δ preferably is greater than about 50° but less than 90°. More preferably, the angular offset δ is more than about 75°. Although the upstream ends Iₐ, I_{b} and downstream ends Oₐ, O_{b} of the seam lines 110 are each disposed about 180° from each other in the illustrated embodiment, it is to be understood that, in additional embodiments, each pair of ends Iₐ, I_{b}, Oₐ, O_{b} can have different angular relationships with one another.

In the embodiment shown in Figures 4-6, the anterior and posterior leaflets 98, 100 are shaped somewhat differently from one another. This allows the valve 90 to have unique aspects such as the saddle-shaped inflow annulus 150. As shown, a width 234 of the anterior leaflet 98 is less than a width 236 of the posterior leaflet 100, but a length 238 of the anterior leaflet 98 is greater than a length 239 of the posterior leaflet 100.

Several valve embodiments can be manufactured by varying the shapes of the flat patterns of the anterior and posterior leaflets. For example, embodiments of various seam line dispositions such as are discussed above with reference to Figures 19-20 can be constructed by varying the flat pattern shapes of the leaflets.

In the illustrated embodiments, the commissural attachment tabs 140 are formed as part of the leaflets 98, 100 and are assembled and connected as discussed above. However, it is to be understood that various types of commissural attachment tabs and various methods for constructing such tabs can be used. For example, commissural attachment tabs can be formed separately from the valve and can be attached to the valve during manufacture.

A notable step when developing prosthetic valves is in vitro testing of a valve prototype. In vitro testing allows developers to predict how the valve will perform in subsequent in vivo testing and in actual use. Of course, the better the in vitro testing apparatus simulates actual heart conditions, the better and more useful the test results.

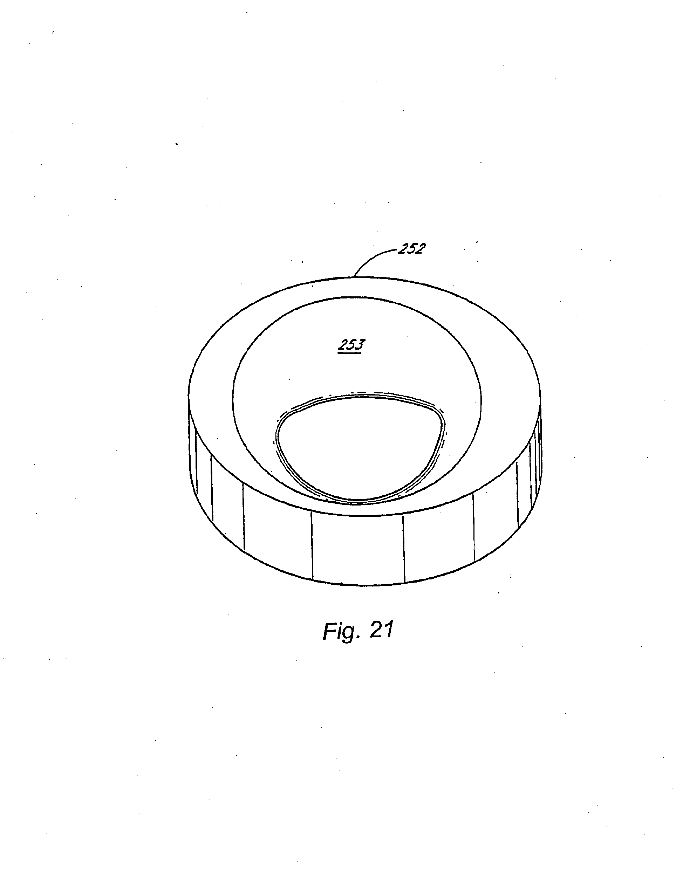

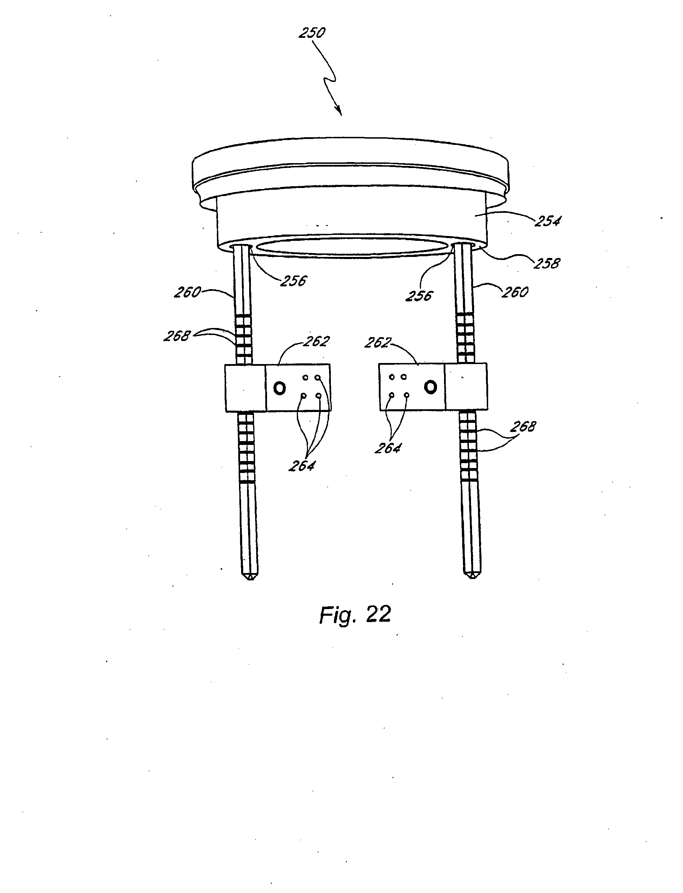

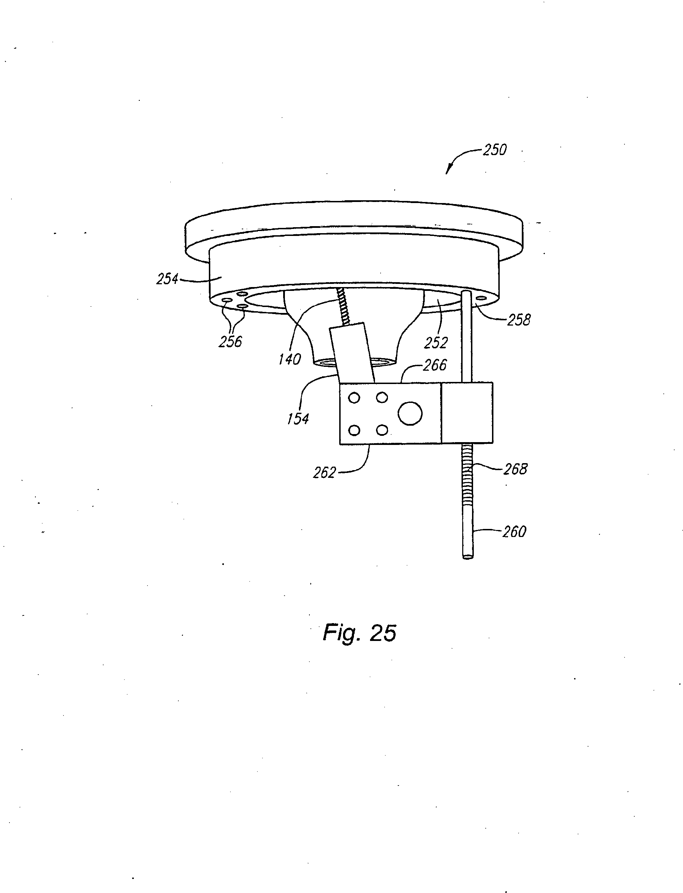

With reference next to Figures 21-25, a prosthetic valve test fixture 250 is provided for in vitro testing of prosthetic mitral valves. The illustrated test fixture is configured to simulate the complex three-dimensional shape and behavior of a human mitral apparatus from which a native valve has been removed. In operation, the test fixture 250 is used in connection with a pulse duplicator (not shown) in order to simulate operation of an actual mitral valve in a pulsing flow of blood.

With specific reference to Figure 21, a simulated mitral annulus 252 has a contoured surface 253 that is generally complementary to the saddle-shaped annulus of a native human mitral valve. In the illustrated embodiment, the simulated valve annulus 252 is cast from a resilient material such as silicone rubber.

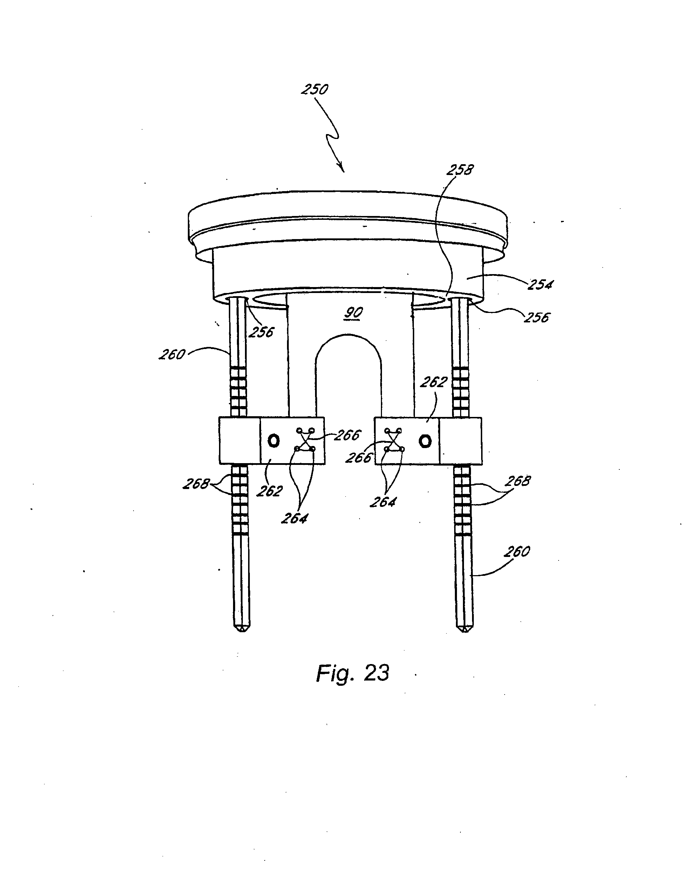

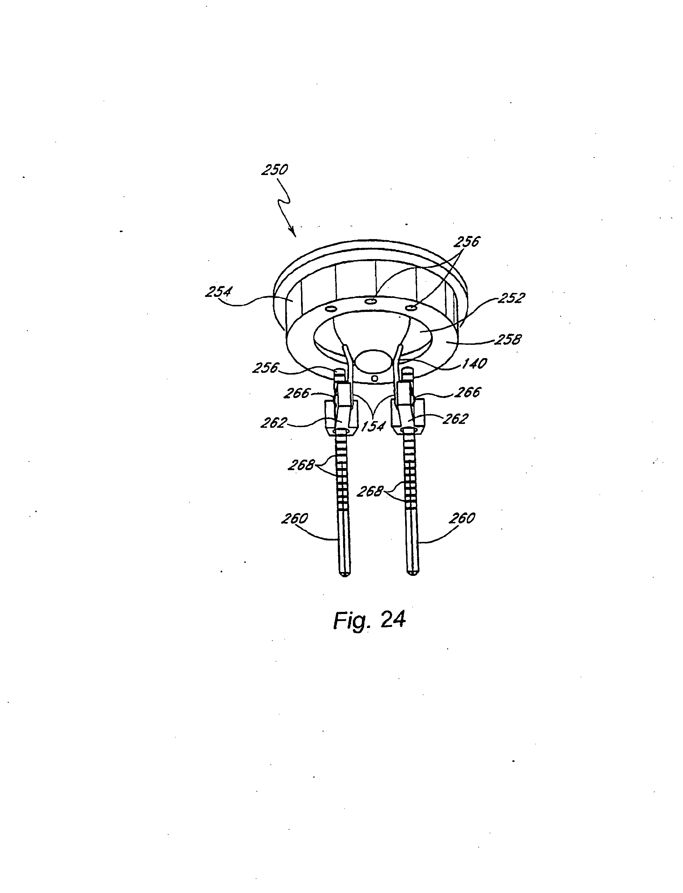

With reference next to Figure 23, a generally ring-shaped base 254 of the test fixture 250 is configured to hold the simulated annulus 252. As shown in Figures 23 and 24, a prosthetic valve 90 to be tested can be mounted in the base 254 with the valve annulus sutured to the simulated annulus 252 and the leaflets 98, 100 of the valve 90 extending through the base 254.

With continued reference to Figures 23-25, threaded holes 256 are formed in a downstream side 258 of the base 252. A pair of rigid rods 260 each have threaded ends that can be selectively threaded into the holes 256 so that the rods 260 are held securely by the holes 256 and extend distally from the base 252. Attachment pads 262 are connected to the rigid rods 260. Suture receiver holes 264 are formed through the attachment pads 262 and are configured so that commissural attachment portions 154 of the prosthetic valve 90 can be attached to the attachment pads 262 with sutures 266. In this manner, the attachment pads 262 simulate papillary muscles and/or chordae tendineae.

The location of the attachment pads 262 relative to the simulated annulus 252 can be controlled by selectively securing the pads 262 at a desired longitudinal position along the rods 260. In the illustrated embodiment, the rods 260 have a series of annular grooves 268 formed therein and the attachment pads 262 have rings that selectively fit into the grooves 268 to hold the pads 262 in place. In additional embodiments, set screws or any type of fastener can be used to hold the attachment pads securely in place relative to the rods.

Figure 23 and 24-25 show the test fixture 250 disposed in different configurations. These figures show different arrangements of the rods 260 relative to the base 254, which results in two methods of holding a prosthetic valve 90 in place. As such, the positioning of the rods 260 is versatile and enables testing of valves having various shapes, sizes and configurations.

The arrangement of the rods 260 depicted in Figures 24-25 enables the valve to be installed in the test fixture 250 in a manner more closely resembling the placement of the valve in a native mitral annulus. For example, papillary muscles extend from the ventricle wall generally on a posterior side of the valve. With the rods 260 disposed on the posterior side of the valve, the valve can be tilted somewhat to better simulate the actual positioning of the valve relative to the simulated annulus 252.

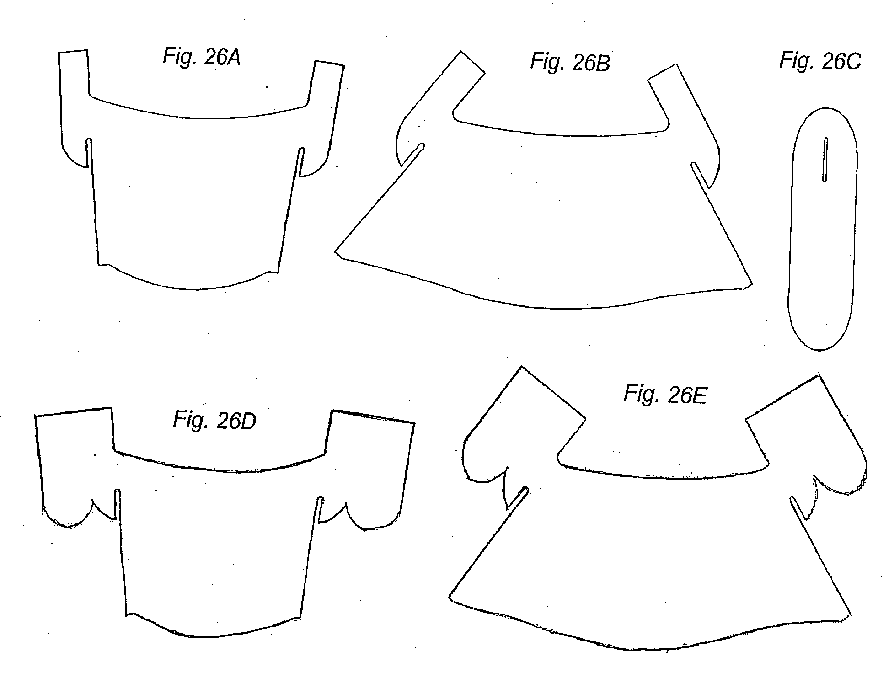

With next reference to Figures 26A-E, flat leaflet patterns are shown. Figure 26A shows an anterior leaflet adapted to be connected to the posterior leaflet shown in Figure 26B to form another embodiment of a replacement mitral valve. Figure 26C shows a connecting portion that is used to connect tab portions of the respective leaflets to one another. Figures 26D and 26E show another method of assembly using only two pieces. In this method of assembly, the connecting portion is incorporated into both the anterior leaflet (Figure 26D) and the posterior leaflet (Figure 26E), creating a two piece assembly.

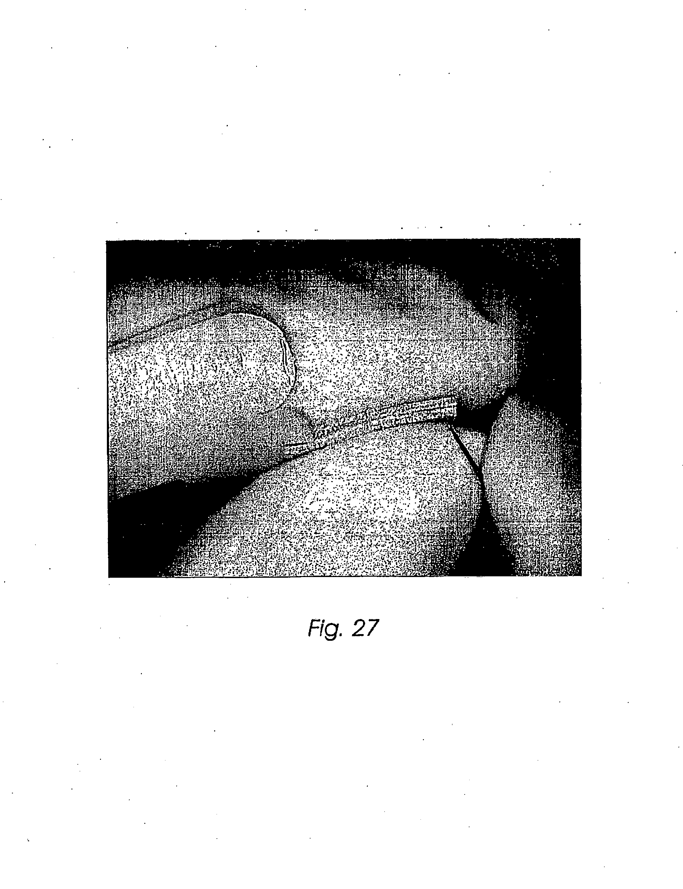

With reference next to Figures 27-44, one embodiment of a method, and the assembly steps for the method, are shown for using the leaflets and connection member of Figures 26A-C to form a replacement mitral valve.

The formation of a replacement mitral valve can be accomplished by following the succeeding assembly steps 6.1-6.19.

Step 6.1 (as shown in Figure 27) instructs the assembler to: Insert a thread through a needle's eye and make a triple loop. To form the first seam line, align anterior and posterior cut edges of leaflets until even. Insert needle through the tissue layers and loop. Pull the stitch tight, and bring the knot above the edge. Place each succeeding stitch at 0.5 mm from the edge with 1.0mm space between stitches. Make a double loop before pulling the stitch up.

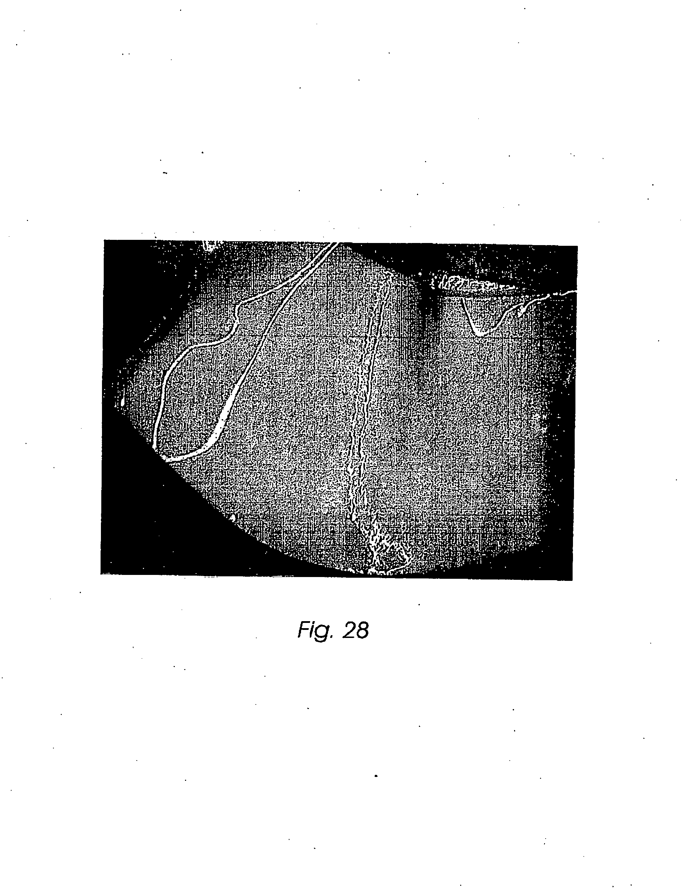

Then, according to Step 6.2 (as shown in Figure 28): Continue to insert the needle and thread until the needle reaches the corner of the tab. Place leaflets inside out and fold two leaflets together. Manipulate them until two cut commissural edges are even. Then sew one more time (duplicate seam-line) until the needle reaches the 1st stitch. Then, according to Step 6.3, repeat Step 6.2 to form the second straight seam line.

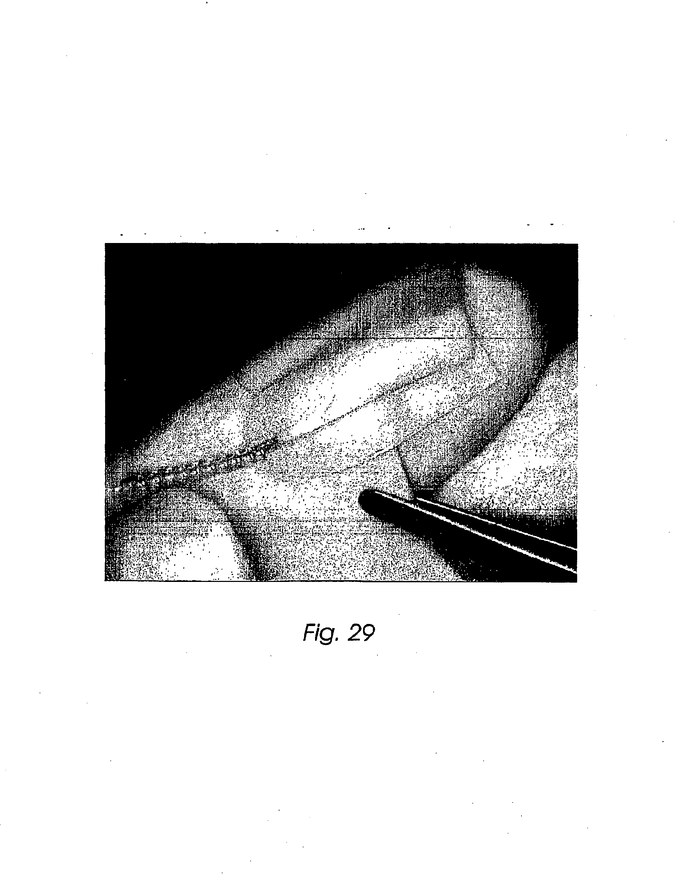

Next, Step 6.4 instructs (as shown in Figure 29): Open the tab. Use forceps to fold back 1/3 from the left tab. Bring the slotted tab toward the commissural coaptation. The slotted tab should be located adjacent to the seam line and behind the other tab.

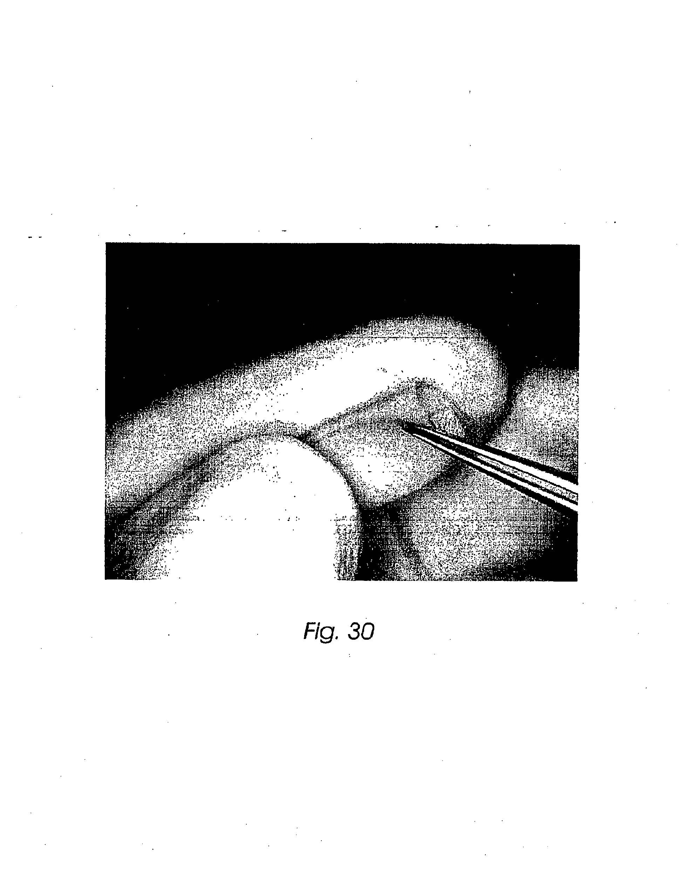

Then, according to Step 6.5 (as shown in Figure 30): Fold 1/3 of the unslotted tab toward the seam line until it overlaps the slotted tab. Check the accuracy of alignment of tab and leaflets to prevent wrinkles and folds. Manipulate the tab until it is evenly centered along the seam line.

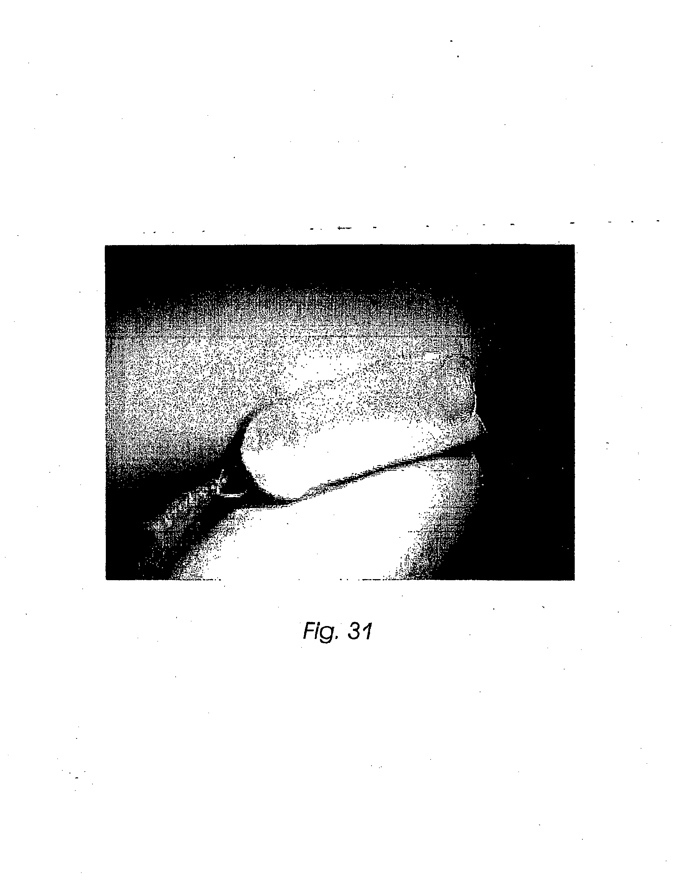

Then, as shown in Figure 31 and explained in Step 6.6: Use 4 stay stitches to keep the tab temporarily in the correct position. The temporary stitches are positioned at these points as shown in the diagram: M'& H, M' & A, M & the left and right adjacent points.

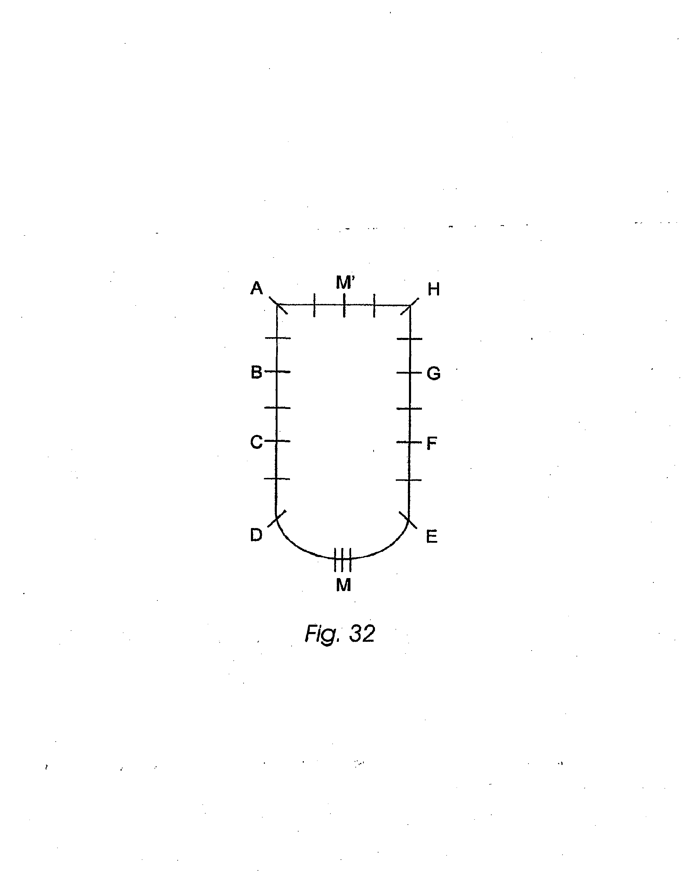

Continuing to Step 6.7, and as shown in Figure 32: Sew the tab as follows, referring to the locations shown on the chart: (a) Insert needle from bottom up (2.0 mm deep from tab end) at midpoint of M' and H and make an overhand double loop knot. Lay thread vertically. (b) Insert needle up (2.0 mm deep) at midpoint of M' and A. Lay thread vertically. (c) Insert needle up (2.0 - 2.25 mm deep) at points B, C, D, E, F and G. At each point make overhand double loop knot. Lay thread vertically.

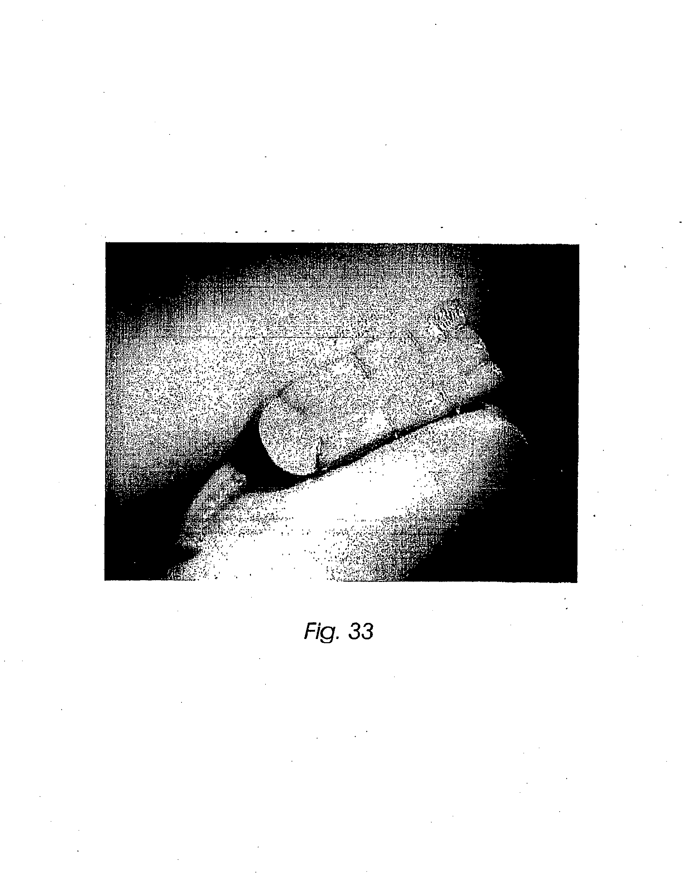

Then, as shown in Figures 33 and Step 6.8, the tissue tab is formed.

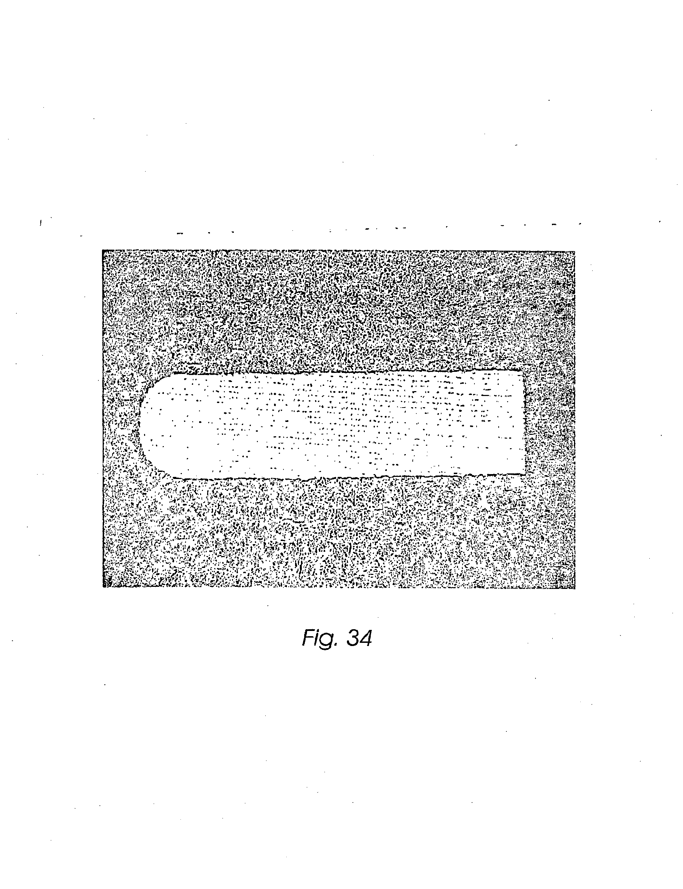

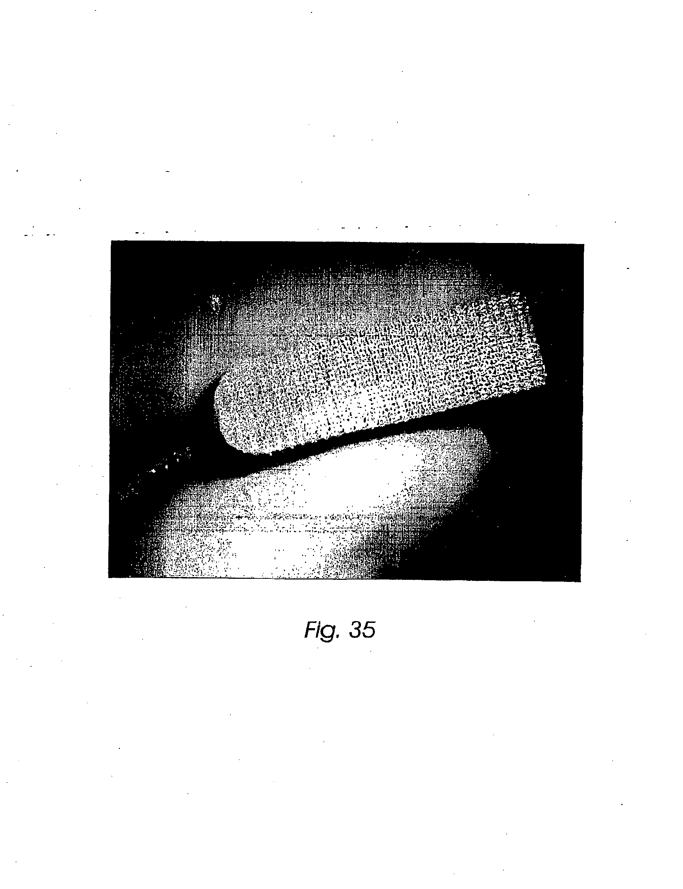

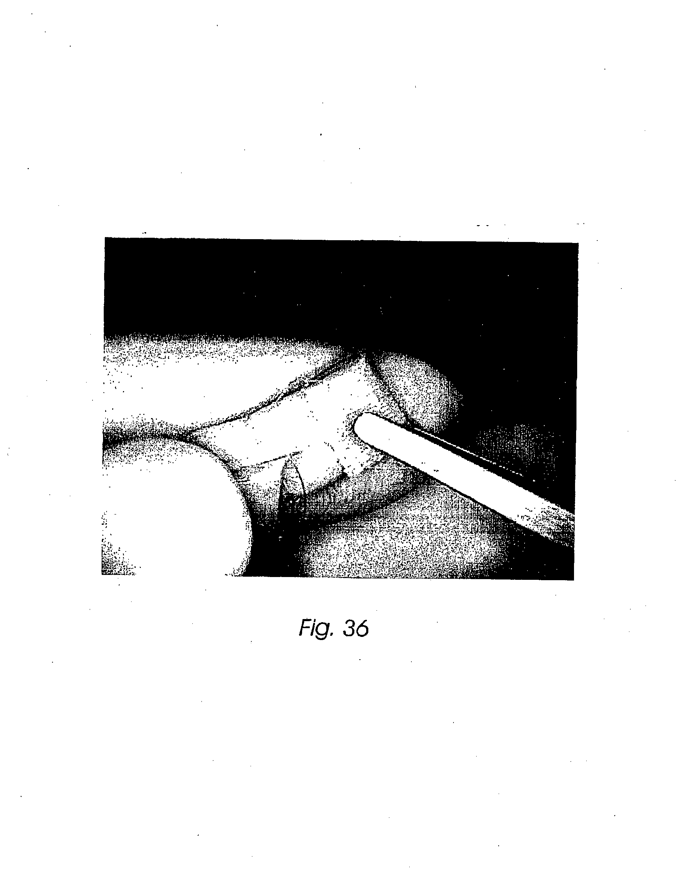

Continuing, and as shown in Figures 34, 35, and 36, in steps 6.9 - 6.11, obtain a reinforcement cloth tab (Step 6.9, Figure 34), align the cloth tab along the surface of the tissue tab (Step 6.10, Figure 35), and fold the cloth tab over the flat end of the tissue tab (Step 6.11, Figure 36).

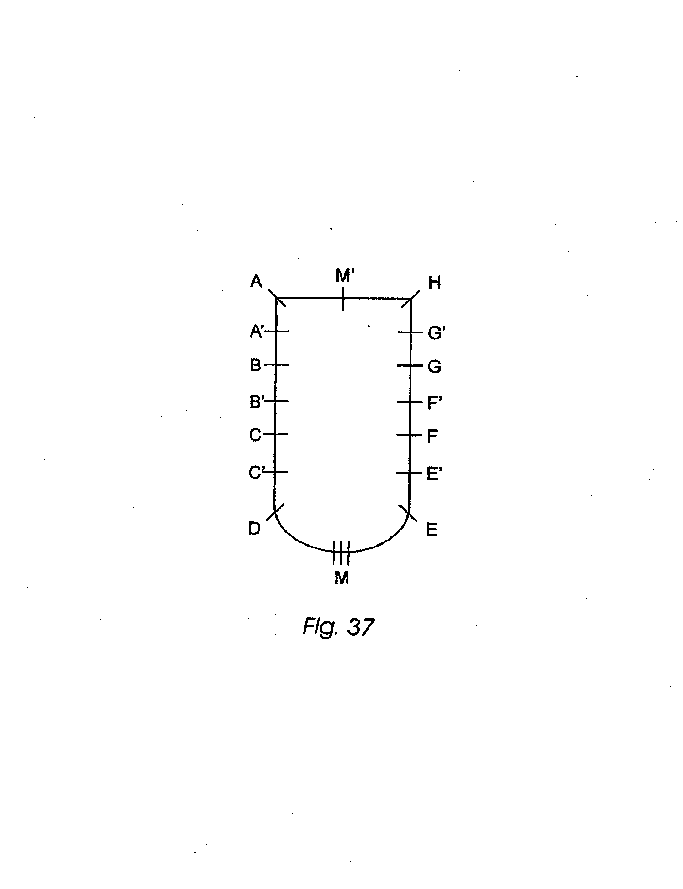

Next, according to Step 6.12 (as shown in Figure 37): Press and stitch through all layers and around the tab. Refer to the locations chart on the right. a) Insert needle (2.0 - 2.25 mm) from bottom up at point G'. Make an overhand loop knot. Lay thread horizontally. b) Insert needle up at point H. Make an overhand loop knot Lay thread diagonally. c) Insert needle up at point M'. Make an overhand loop knot. Lay thread vertically. d) Insert needle up at point A. Make an overhand loop knot. Lay thread diagonally. e) Insert needle at points A', B, B',C, and C' and make an overhand loop knot at each point. Lay thread horizontally. f) Insert needle up at point D. Make an overhand loop knot. Lay thread diagonally. g) Insert needle up (1.0 mm -1.5 mm) from the bottom of tab adjacent the point left of M. Make an overhand loop knot. Lay thread vertically. h) Insert Needle up (1.0 mm - 1.5 mm) from the bottom of tab to the point adjacent and right of point M. Make an overhand loop knot. Lay thread vertically. i) Insert needle up at point E. Make an overhand loop knot. Lay thread diagonally. j) Insert the needle and stitch from points E',F,F', and G. At each point make an overhand loop knot. Lay thread horizontally. k) Insert the needle and stitch from point G'. Lay thread horizontally. Make a triple loop knot. Hide knot inside the cloth tab before cutting the suture. Note: At points B, C, D, E, E and G, after aligning the cloth tab with the tissue tab, insert needle up at the same hole.

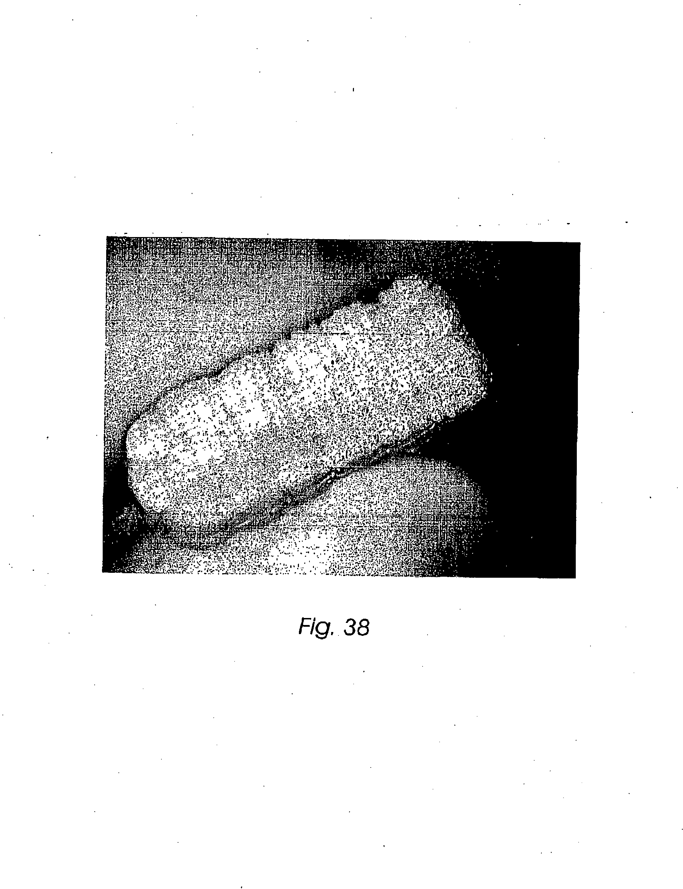

Then, as shown in Figure 38 in Step 6.13, the tab is formed.

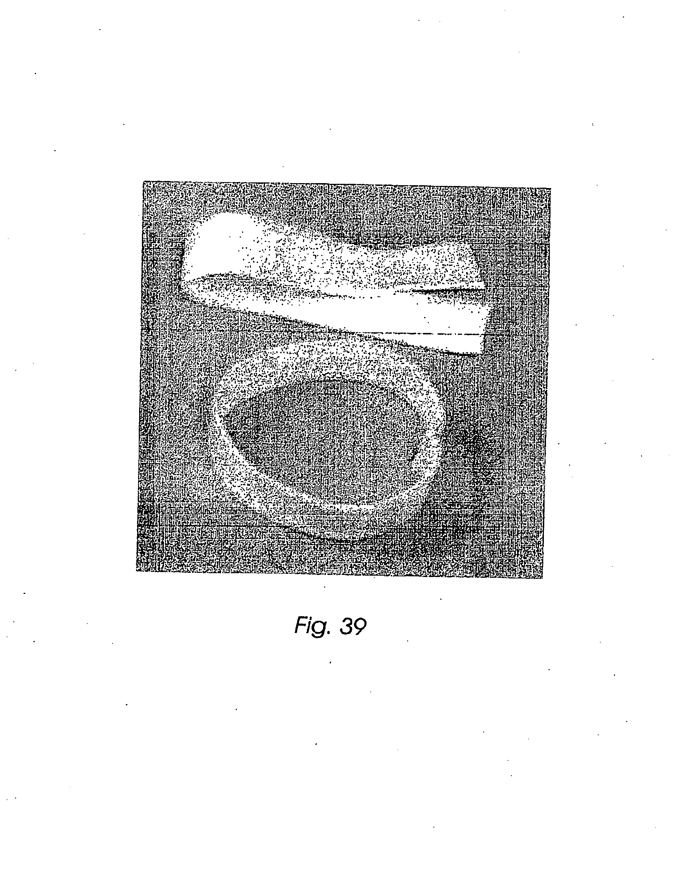

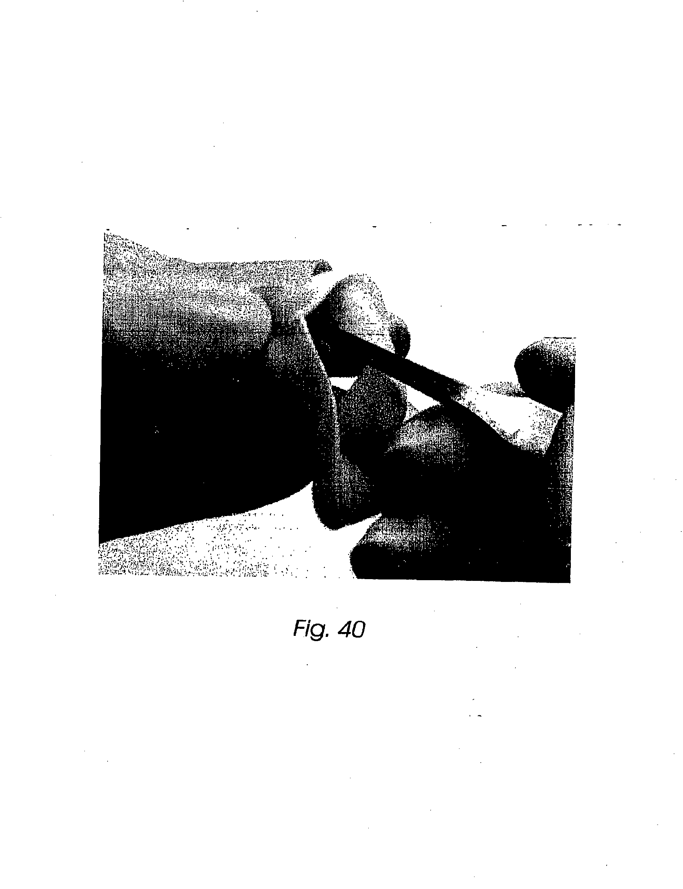

Next, and as shown in Figures 39 and 40, and explained in Steps 6.14 and 6.15, Sew the ends of a sewing ring tape together (0.5mm from each end) to form a closed ring (Step 6.14, Figure 39), and wrap sewing ring along the inflow edge of the apparatus. The seam of the sewing ring aligned with one seam line of the leaflet joints (Step 6.15, Figure 40).

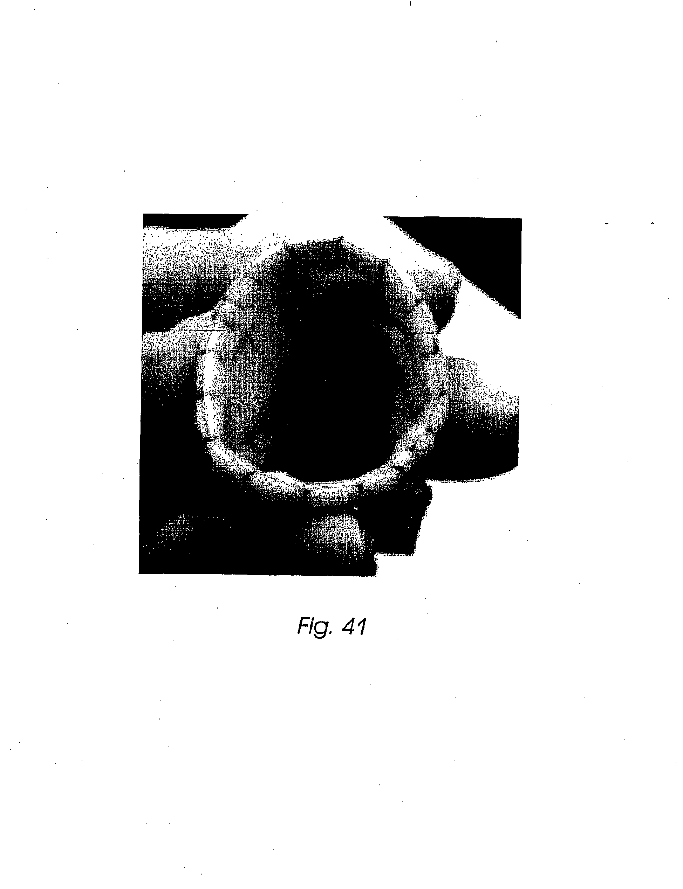

Then, Step 6.16 (as shown in Figure 41) explains: Use basting stitches to temporarily hold the sewing ring in place. Then use running stitches for fitting and construction. Make two vertical marker stitches on the anterior leaflet and one vertical marker stitch at the center of the posterior leaflet. Cut and remove the basting stitches using scissors and forceps.

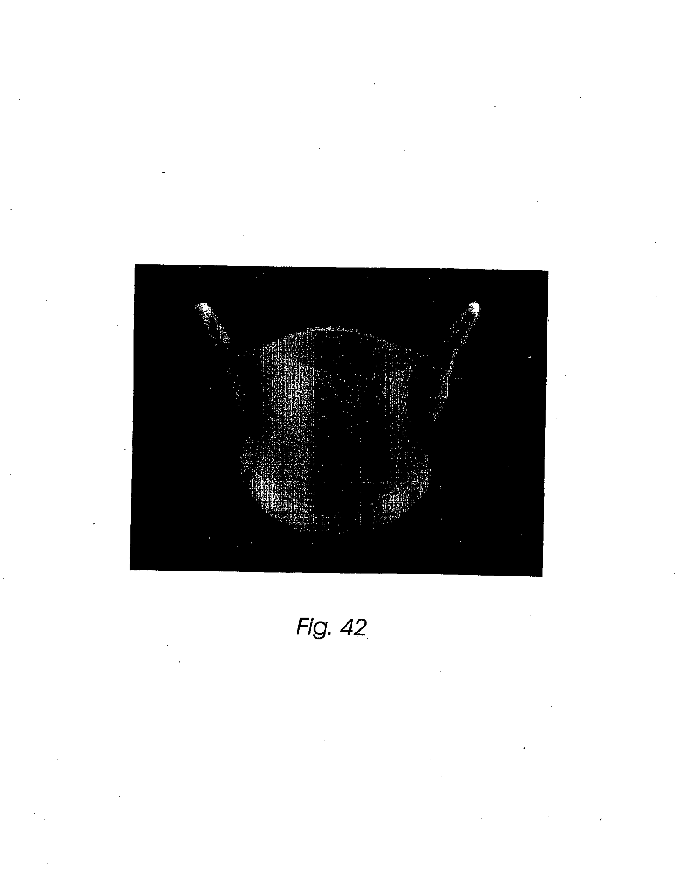

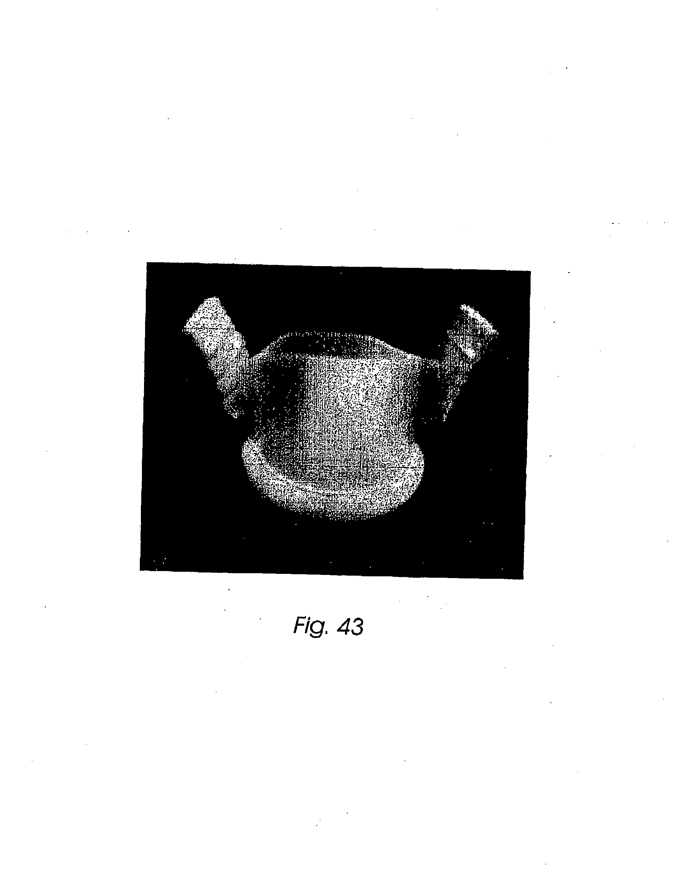

The, according to Steps 6.17 - 6.18 (as shown in Figures 42 and 43), the two anterior marker stitches and the single posterior marker stitch are completed.

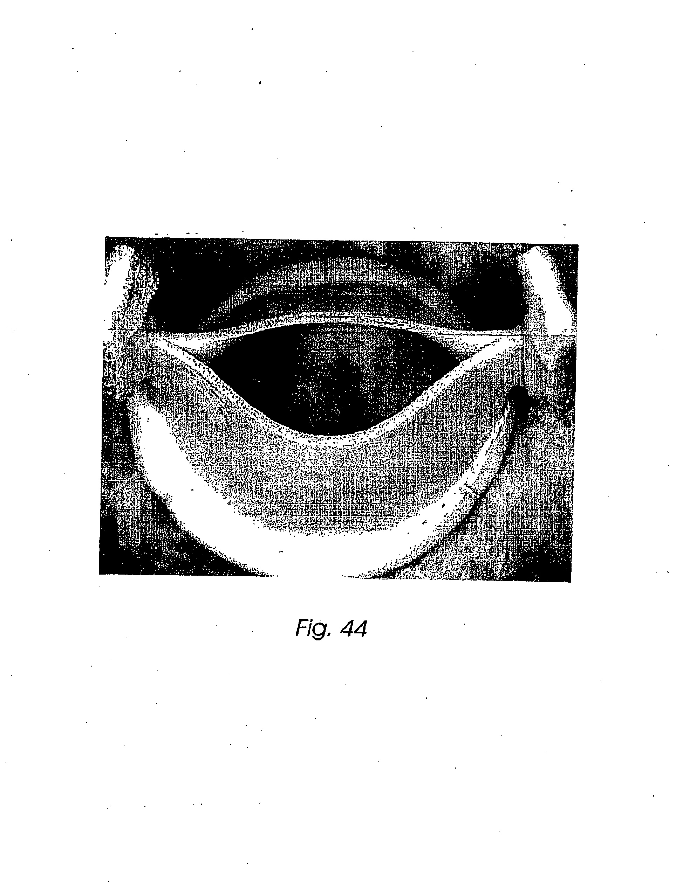

Finally, as specified in Step 6.19 (as shown in Figure 44): Place the assembled apparatus in fixation solution in a closed container and store it under refrigeration until next operation.

Although the enclosed document specifies certain specific materials, it is to be understood that, in other embodiments, substitutions can be made and/or specific steps and materials may be eliminated or added. Additionally, it is anticipated that all or some of the materials can be included together in a kit.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In addition, while a number of variations of the invention have been shown and described in detail, other modifications, which are within the scope of this invention, will be readily apparent to those of skill in the art based upon this disclosure. It is also contemplated that various combinations or subcombinations of the specific features and aspects of the embodiments may be made and still fall within the scope of the invention. Accordingly, it should be understood that various features and aspects of the disclosed embodiments can be combined with or substituted for one another in order to form varying modes of the disclosed invention. Thus, it is intended that the scope of the present invention herein disclosed should not be limited by the particular disclosed embodiments described above, but should be determined only by a fair reading of the claims that follow.

## Claims

1. A prosthetic atrioventricular replacement valve, comprising :
a valve body (92) having an anterior leaflet (98) and a posterior leaflet (100), the leaflets (98, 100) connected to each other along hinge lines (144), an inlet portion (94) comprising an annulus (150) and an outlet portion (96) having at least two commissural attachment locations (154), **characterized in that** said annulus (150) has a periphery (160), the edges of said periphery being scalloped and saddle-shaped as defined by a pair of relatively high peripheral portions (162, 164) separated by a pair of relatively low peripheral portions (174, 176).

2. The valve of Claim 1, wherein one of the high peripheral portions (162) is higher than the other of the high peripheral portions (164).

3. The valve of Claim 2, wherein the annulus (150) has an annulus tilt angle in the range of 12-20 degrees.

4. The valve of Claim 3, wherein the annulus (150) has a shape which is non-circular when viewed in a direction perpendicular to the plane of the annulus (204).

5. The valve of Claim 4, wherein said non-circular shape is generally on oval shape.

6. The valve of Claim 5, wherein said oval shape has a major axis (192) extending between the low portions and a minor axis (190) extending between the high portions.

7. The valve of Claim 6, wherein said oval shape is asymmetric with respect to at least one of said major and minor axes (192, 190).

8. The valve of Claim 7, wherein the annulus has a generally ovoid shape.

9. The valve of Claim 6, wherein said oval shape is symmetric with respect to said minor axis (190).

10. The valve of Claim 1, wherein said valve body (92) comprises a pair of hinge lines (144) at which said body preferentially bends to form an anterior leaflet (98) and a posterior leaflet (100).

11. The valve of Claim 10, wherein the hinge lines (144) are disposed so that at least the portion of the anterior leaflet (98) adjacent said annulus (150) subtends significantly less than 180° of said annulus.

12. The valve of Claim 10, wherein the hinge lines (144) are formed by respective seams (110) extending longitudinally along the valve body (92).

13. The valve of Claim 12, wherein the seams (110) are formed by stitching an interior side of the posterior leaflet (100) in facing relationship with an interior side of the anterior leaflet (98), whereby said seams (110) provide a slight biasing of the leaflets (98, 100) towards each other to aid in closing of the valve, without significantly restricting fluid flow from the annulus (150) through the valve body (92) when the valve is open.

14. The valve of Claim 10, wherein the hinge lines (144) are disposed such that, upon closure of the valve, the commissural line between the leaflets (98, 100) is curved substantially towards the anterior side of the valve, whereby the anterior leaflet (98) forms a trough through which blood flows from the ventricle to the aorta.

15. A method of manufacturing a prosthetic replacement atrioventricular valve according to any of the preceding claims, comprising: providing a sheet of tissue; and
cutting an anterior leaflet (98) and posterior leaflet (100) from said tissue, said cutting comprising cutting an inflow end of the anterior leaflet (98) on a radius of curvature different than that of an inflow end of the posterior leaflet (100).

## Patentansprüche

1. Künstliche atrioventrikuläre Ersatzklappe, umfassend:
einen Klappenkörper (92) mit einem vorderen Klappensegel (98) und einem hinteren Klappensegel (100), wobei die Klappensegel (98, 100) miteinander entlang von Faltlinien (144) verbunden sind, einen Einlassbereich (94), der einen Ring (150) aufweist, und einen Auslassbereich (96) mit mindestens zwei Kommissurbefestigungsstellen (154), **dadurch gekennzeichnet, dass** der Ring (150) eine Umfangslänge (160) aufweist, wobei die Ränder der Umfangslänge gewellt und sattelförmig sind, wie durch ein Paar relativ hohe Umfangsabschnitte (162, 164) definiert, die durch ein Paar relativ niedrige Umfangsabschnitte (174, 176) getrennt sind.

2. Klappe nach Anspruch 1, wobei einer der hohen Umfangsabschnitte (162) höher als der andere der hohen Umfangsabschnitte (164) ist.

3. Klappe nach Anspruch 2, wobei der Ring (150) einen Ring-Neigungswinkel in dem Bereich von 12 - 20 Grad aufweist.

4. Klappe nach Anspruch 3, wobei der Ring (150) eine Form aufweist, die nicht kreisförmig ist, wenn er in einer Richtung betrachtet wird, die senkrecht zu der Ebene des Ringes (204) ist.

5. Klappe nach Anspruch 4, wobei die nicht-kreisförmige Form im Allgemeinen eine ovale Form ist.

6. Klappe nach Anspruch 5, wobei die ovale Form eine Hauptachse (192), die sich zwischen den niedrigen Abschnitten erstreckt, und eine Nebenachse (190), die sich zwischen den hohen Abschnitten erstreckt, aufweist.

7. Klappe nach Anspruch 6, wobei die ovale Form in Bezug auf mindestens eine von den Haupt- und Nebenachsen (192, 190) asymmetrisch ist.

8. Klappe nach Anspruch 7, wobei der Ring eine im Allgemeinen eiförmige Form aufweist.

9. Klappe nach Anspruch 6, wobei die ovale Form in Bezug auf die Nebenachse (190) symmetrisch ist.

10. Klappe nach Anspruch 1, wobei der Klappenkörper (92) ein Paar Faltlinien (144) umfasst, an denen der Körper sich bevorzugt biegt, um ein vorderes Klappensegel (98) und ein hinteres Klappensegel (100) zu bilden.

11. Klappe nach Anspruch 10, wobei die Faltlinien (144) derart angeordnet sind, dass mindestens der Abschnitt des vorderen Klappensegels (98), der benachbart zu dem Ring (150) liegt, sich über deutlich weniger als 180° des Ringes erstreckt.

12. Klappe nach Anspruch 10, wobei die Faltlinien (144) durch die entsprechenden Nähte (110) gebildet sind, die sich in Längsrichtung entlang des Klappenkörpers (92) erstrecken.

13. Klappe nach Anspruch 12, wobei die Nähte (110) durch Vernähen einer Innenseite des hinteren Klappensegels (100) mit einer zugewandten Innenseite des vorderen Klappensegels (98) gebildet werden, wobei die Nähte (110) eine leichte Neigung der Klappensegel (98, 100) aufeinander zu vorsehen, um das Schließen der Klappe zu unterstützen, ohne die Fluidströmung von dem Ring (150) durch den Klappenkörper (92) signifikant einzuschränken, wenn die Klappe offen ist.

14. Klappe nach Anspruch 10, wobei die Faltlinien (144) derart angeordnet sind, dass durch das Schließen der Klappe, die Kommissurlinie zwischen den Klappensegeln (98, 100) im Wesentlichen in Richtung der vorderen Seite der Klappe gekrümmt ist, wodurch das vordere Klappensegel (98) eine Vertiefung bildet, durch die das Blut vom Vorhof zur Aorta strömt.

15. Verfahren zur Herstellung einer künstlichen arterioventrikulären Ersatzklappe gemäß jedem der vorhergehenden Ansprüche, umfassend: Bereitstellen eines Gewebestücks; und
Zuschneiden eines vorderen Klappensegels (98) und eines hinteren Klappensegels (100) aus dem Gewebe, wobei das Zuschneiden das Zuschneiden von einem Zuflussende des vorderen Klappensegels (98) mit einem Krümmungsradius umfasst, der unterschiedlich ist zu einem Zuflussende des hinteren Klappensegels (100).

## Revendications

1. Valve prothétique atrioventriculaire de remplacement, comprenant :
un corps de valve (92) doté d'un feuillet antérieur (98) et d'un feuillet postérieur (100), les feuillets (98, 100) étant connectés l'un à l'autre le long de lignes de charnières (144), une section d'admission (94) comprenant un anneau (150) et une section de sortie (96) ayant au moins deux sites de fixation des commissures (154), **caractérisée en ce que** ledit anneau (150) a une périphérie (160), les bords de ladite périphérie étant crantés et en forme de selles, telle que définie par une paire de sections périphériques relativement élevées (162, 164) séparées par une paire de sections périphériques relativement basses (174, 176) .

2. Valve selon la revendication 1, dans laquelle une des sections périphériques élevées (162) est plus haute que l'autre section périphérique élevée (164).

3. Valve selon la revendication 2, dans laquelle l'anneau (150) a un angle d'inclinaison d' anneau de l'ordre de 12 à 20 degrés.

4. Valve selon la revendication 3, dans laquelle l'anneau (150) a une forme non circulaire vu dans un sens perpendiculaire au plan de l'anneau (204).

5. Valve selon la revendication 4, dans laquelle ladite forme non circulaire est généralement une forme ovale.

6. Valve selon la revendication 5, dans laquelle ladite forme ovale a un axe majeur (192) s'étendant entre les sections basses et un axe mineur (190) s'étendant entre les sections élevées.

7. Valve selon la revendication 6, dans laquelle ladite forme ovale est asymétrique par rapport à au moins un desdits axes majeur et mineur (192, 190).

8. Valve selon la revendication 7, dans laquelle l'anneau (162) a une forme globalement ovoïde.

9. Valve selon la revendication 6, dans laquelle ladite forme ovale est symétrique par rapport audit axe mineur (190).

10. Valve selon la revendication 1, dans laquelle ledit corps de valve (92) comprend une paire de lignes de charnières (144) sur lequel ledit corps se courbe préférentiellement pour former un feuillet antérieur (98) et un feuillet postérieur (100).

11. Valve selon la revendication 10, dans laquelle les lignes de charnières (144) sont disposées de manière à ce qu'au moins la section du feuillet antérieur (98) adjacente audit anneau (150) sous-tende nettement moins de 180° dudit anneau.

12. Valve selon la revendication 10, dans laquelle les lignes de charnières (144) sont formées par des coutures respectives (110) s'étendant longitudinalement le long du corps de valve (92).

13. Valve selon la revendication 10, dans laquelle les coutures (110) sont formées en cousant une face interne du feuillet postérieur (100) face à face avec une face intérieure du feuillet antérieur (98), lesdites coutures (110) créant une légère inclinaison (98, 100) des feuillets l'un vers l'autre afin d'aider à la fermeture de la valve sans restreindre significativement l'écoulement de fluide depuis l'anneau (150) à travers le corps de valve (92) lorsque la valve est ouverte.

14. Valve selon la revendication 10, dans laquelle les lignes de charnières (144) sont disposées de manière à ce que, à la fermeture de la valve, la ligne de commissure entre les feuillets (98, 100) soit courbée sensiblement vers la face antérieure de la valve, ce qui a pour effet que le feuillet antérieur (98) forme une cuvette à travers laquelle le sang circule du ventricule vers l'aorte.

15. Procédé de fabrication d'une valve prothétique atrioventriculaire de remplacement selon l'une quelconque des revendications précédentes, consistant à : se procurer une feuille de tissu et
découper un feuillet antérieur (98) et un feuillet postérieur (100) dans ledit tissu, ledit découpage comprenant la découpe d'une extrémité d'afflux du feuillet antérieur (98) sur un rayon de courbure différent de celui d'une extrémité d'afflux du feuillet postérieur (100).
